# EUROPEAN PATENT SPECIFICATION

(11) **EP 3 864 014 B1**
(45) Date of publication and mention of the grant of the patent: **05.07.2023**
(21) Application number: 19790887.4
(22) Date of filing: 09.10.2019
(51) Int. Cl.: C07D 417/14, A61K 31/517, A61P 35/00

(54) **MALONATE SALT OF VARLITINIB**
MALONATSALZ VON VARLITINIB
SEL DE MALONATE DE VARLITINIB

(30) Priority: 09.10.2018 SG 10201808900X
(43) Date of publication of application: 18.08.2021
(73) Proprietor: ASLAN Pharmaceuticals Pte Ltd, UE Square 239920 (SG)
(72) Inventor: MOORE, Robert, Singapore 089824 (SG)
(74) Representative: Sterling IP
(86) International application number: PCT/SG2019/050504
(87) International publication number: WO 2020/076239

(56) References cited:
- WO-A1-2005/016346
- WO-A1-2017/037299

## Description

The present disclosure relates to a pharmaceutically acceptable salt of compounds of formula (I), such as varlitinib, a method of producing the salt, a purer form of the free base obtainable from the salt, and a pharmaceutical composition comprising any one of the same. Also provided is a salt, free base or composition thereof for use in treatment, in particular the treatment of cancer, including as part of a combination therapy, for example in combination with a chemotherapeutic agent.

The disclosure also extends to novel polymorphic forms, compositions comprising the same and use thereof in treatment, in particular treatment of cancer. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### BACKGROUND

There are many cancers that are difficult to treat and although therapy is available, there appears to exist or to come into existence, a degree of resistance to the therapy. Primary resistance may occur in that cancer does not respond to treatment from the outset. This is may be due to the fact that the therapies used do not target precisely enough and effectively enough the specific type of cancer. Secondary or acquired resistance also occurs quite frequently, which means that a therapy to which initially the patient seems to respond over time loses its efficacy. With chemotherapy, for example, overall approximately 80% of patients diagnosed with ovarian epithelial, primary peritoneal cancer will relapse after first-line platinum-based and taxane-based chemotherapy. Treatment of hepatocellular carcinoma with chemotherapy has a reported response rate of 10-20%. These are not very encouraging statistics.

There are numerous reasons for resistance, for example some cancers are discovered at a late stage and/or a simply not responsive to treatment

Mechanisms by which cancers avoid the therapeutic effect of therapy include but are not limited to:
i) mutations which render the cancer less vulnerable to the treatment (e.g. mutation of the site of action of the therapy),
ii) active transportation of the drug out of the tumor, for example by p-glycolation,
iii) building up physical defences, for example stroma and tumor associated macrophages which inhibit certain immune responses, and
iv) certain cancers develop paths to repair damage caused by some anti-cancer therapies.

Tumor heterogeneity may also contribute to resistance, where small subpopulations of cells may acquire or stochastically already possess some of the features enabling them to emerge under selective drug pressure (for example through mechanisms, such as cancer stem cells). Thus even if a number or even a majority of the cancer cells are treated then resistant cells go into hibernation and then re-emerge at a later date. This is a problem that many patients with cancer encounter, and it obviously limits the therapeutic alternatives that are effective and worsens the prognosis.

Cancer therapy guidelines describe recommended cancer therapies, which includes recommendations for the order or sequence in which the therapies are employed. Thus, if a patients show disease progression on the first therapy ("first line"), then a next therapy ("second line") is recommended, and so on. These therapy recommendations are based on available scientific data and experience, and illustrate that resistance to one therapy does not exclude that another therapy may be effective. At a late stage cancers do not respond to most therapies and no more avenues of therapy exist. These cancers are completely therapy refractory, unless new therapies can be found which are effective.

Cholangiocarcinoma is a prime example of both primary and secondary resistance and is considered to be an incurable and a rapidly lethal malignancy unless both the primary tumor and any metastases can be fully resected (removed surgically). No curative treatment exists for cholangiocarcinoma except surgery. Unfortunately, most patients have advanced stage disease which is inoperable at the time of diagnosis. Patients with cholangiocarcinoma are generally managed - though never cured - with chemotherapy, radiation therapy, and other palliative care measures. These are also used as adjuvant therapies (i.e. post-surgery treatment) in cases where resection has apparently been successful (or nearly so).

In the Western hemisphere cholangiocarcinoma is a relatively rare neoplasm that is classified as an adenocarcinoma (a cancer that forms glands or secretes significant amounts of mucins). It has an annual incidence rate of 1-2 cases per 100,000 in the Western world. However, rates of cholangiocarcinoma have been rising worldwide over the past several decades. Furthermore, the incidence is higher in Asian countries where it is recognized as a significant problem.

(R)-N4-[3-Chloro-4-(thiazol-2-ylmethoxy)-phenyl]-N6-(4-methyl-4,5,-dihydro-oxazol-2-yl)-quinazoline-4,6-diamine (varlitinib also known as ASLAN001 and ARRY-543) (see Example 52 disclosed in WO2005/016346), is a small-molecule pan-HER inhibitor. It is a potentially interesting and effective therapeutic agent.

Varlitinib is prepared in the prior art by reacting a thiourea core with an aniline head-group as summarized in the scheme below:

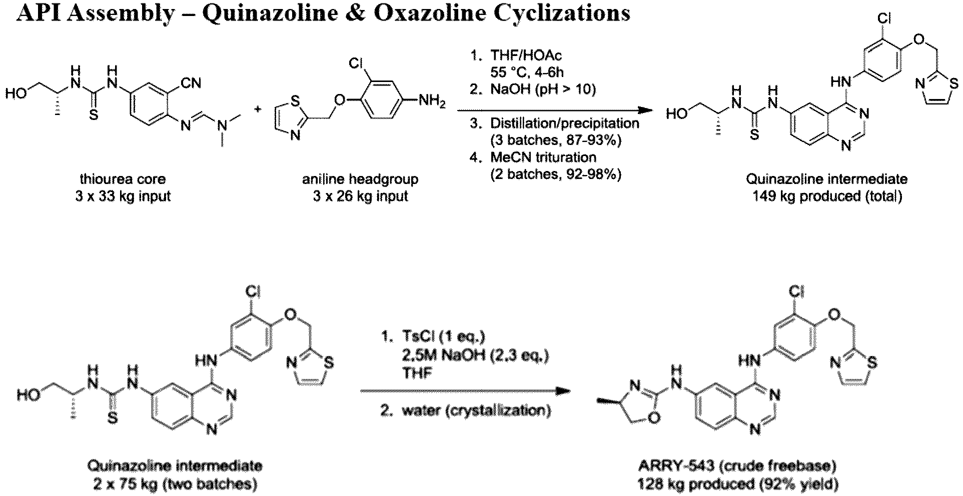

This crude base material is referred to herein as stage 7 material.

However, varlitinib is difficult to manufacture and purify for the reasons discussed below. The free base material of stage 7 is crystalline. However, recrystallisation does not result in material of adequate purity. In commercial processes, filtering and washing may be used to isolate the compound. However, varlitinib free base is difficult to filter because it clogs filters, as it either tends to form very fine small needles or microcrystalline material, which restricts liquid flow even under nitrogen pressure and/or vacuum.

Preparative HPLC can be used to purify compounds but the process is slow and prohibitively expensive when a large amount of compound is required. In addition prep-HPLC requires use of large amounts of solvents, which then have to be disposed of safely. Thus it is simply not commercially viable to employ preparative HPLC to purify large quantities of an active pharmaceutical ingredient (API). It would be better for patients if the compound could be made on a commercial scale with higher levels of purity, because first it would minimise the content of undesirable impurities and potentially allows the therapy to be employed in non-cancer patients and/or prophylactically.

At the present time the manufacturing process for varlitinib includes a further two steps, namely conversion to the ditosylate salt (referred to herein as "Stage 8") and then conversion back to the free base (referred to herein as "Stage 9"), this results in material that is about 97% pure. This material is referred to herein as stage 9 material. However, it is desirable to minimise further one or two impurities that are present in this stage 9 material, for example the aniline impurity and/or the thiazoline impurity. As for the Stage 8 material, this has a high molecular weight, which makes it less clinically desirable and the presence of the strong tosic acid may result in an unacceptable stability profile.

WO2017/037299 relates to the treatment of biliary cancer by administering valitinib.

Accordingly, there is a further need for improved versions of the Stage 8 and Stage 9 materials.

### SUMMARY OF THE DISCLOSURE

The present inventors have surprisingly found that vartlitinib malonate has good balance of pharmaceutically acceptable properties and can be produced on a commercial scale with reduced levels of at least one impurity, for example the aniline impurity.

Advantageously, the varlitinib salt of the present disclosure is crystalline, the crystal form is not needles, it has good physical and chemical stability, for example it has a low propensity to form hydrates, and it has adequate solubility at gastric pHs. The ratio of counter-ion to base is 1:1, which is uncomplicated and the overall purity of the salt is generally at similar or higher levels to the free base from current stage 9, but generally with reduced levels of the aniline and/or thiazoline impurities.

Thus, the presently claimed salt has a high purity, high stability, and can be produced on a commercial scale. Furthermore, the malonate salt may also be easier to filter and handle than the free base of the compound of formula (I). Furthermore, the salt is thought to be safe for use in human therapy, for example due to the reduced levels of at least one impurity, such as the aniline purity. Furthermore, the free base released from the malonate has corresponding reduced levels of at the aniline impurity which previously could not be removed on a commercial scale. This purer version of the free base can be used as the therapeutic agent or may be converted into a different pharmaceutically acceptable salt

The invention will be summarised in the following paragraphs:
1. A pharmaceutically acceptable salt of a compound of formula (**I**): 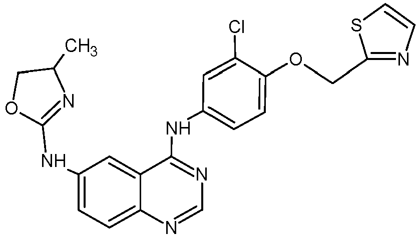 or an enantiomer thereof, wherein the salt is a malonate salt
2. The salt according to paragraph 1, wherein the compound of formula (I) is: 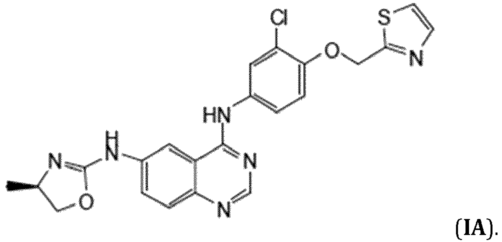
3. The salt according to any one of paragraphs 1 or 2, wherein the purity of the salt is 97% or greater, such as 97.5%, 98%, 98.5%, 99% or 99.5%, for example when analysed by HPLC or similar techniques and expressed as an area/area value (%area).
4. The salt according to any one of paragraphs 1 to 3, wherein salt contains 0.1 % or less of an aniline impurity C₁₈H₁₄ClN₅OS, for example when analysed by HPLC or similar techniques and expressed as an area/area value.
5. The salt according to paragraph 4, wherein the aniline impurity has the structure of formula **(II):** 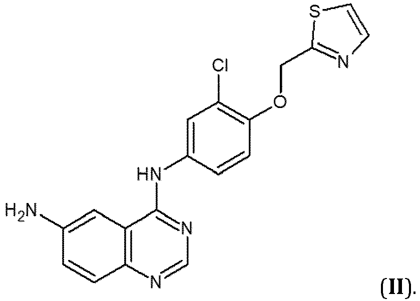
6. The salt according to paragraphs 4 or 5, wherein said aniline impurity is present at 0.09% or less, for example 0.08%, 0.06%, 0.05 or less.
7. The salt according to paragraph 6, wherein said aniline impurity is present at 0.04, 0.03, 0.02, 0.01% or less, in particular 0.02% or less.
8. The salt according to any one of paragraphs 1 to 7, wherein the salt contains 1.5% or less, for example 1.5%, 1.4%, 1.3%, 1.2%, 1.1%, 1%, 0.9%, 0.8%, 0.7%, 0.6%, 0.5% or less of a thiazoline impurity C₂₂H₁₉ClN₆OS₂, for example when analysed by HPLC or similar techniques and expressed as an area/area value (%area).
9. The salt according to paragaph 8, wherein thiazoline impurity has the structure of formula (**III**) 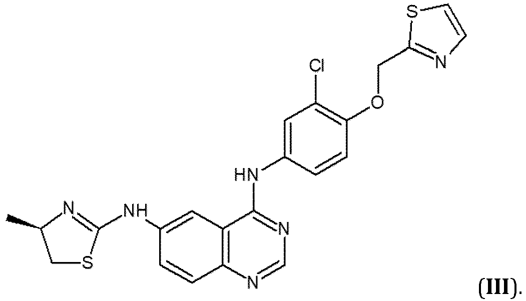
10. A pharmaceutical composition comprising a salt according to any one of paragraphs 1 to 9.
11. A pharmaceutical composition according to paragraph 12, provided as one or more unit doses, for example one or more tablets or capsules.
12. A pharmaceutical composition according to paragraph 12 or 13, wherein the composition is for oral administration.
13. A pharmaceutical composition according to any one of paragraphs 12 to 14, wherein the dose of free base equivalent provided by the malonate salt is in the range 100mg to 900mg, such as 200mg to 500mg, in particular 200mg, 300mg, or 400mg.
14. A salt according to any one of paragraphs 1 to 11 or a pharmaceutical composition according to any one of claims 12 to 15 for use in treatment.
15. A salt or pharmaceutical composition according to paragraph 14 for use in treating cancer, for example an epithelial cancer.
16. Use of a salt according to any one of paragraphs 1 to 9 or a composition according to any one of paragraphs 10 to 13, for the manufacture of a medicament for the treatment of cancer.
17. A salt, composition or use according to any one of paragraphs 14 to 16, wherein the salt of formula (**I**) is administered bi-daily.
18. A salt, composition or use according to any one of paragraphs 14 to 17, wherein the therapy comprises a further therapeutic agent.
19. A salt, composition or use according to paragraph 18, wherein the further therapeutic agent is an anticancer agent
20. A salt, composition, method or use according to paragraph 18 or 19, wherein the anticancer agent is a biological therapeutic agent, such as an antibody molecule.
21. A salt, composition, method or use according to paragraph 20, wherein the biological therapeutic agent is an antibody molecule selected from the group comprising a PD-1 inhibitor, a PD-L1 inhibitor, a CTLA-4 inhibitor, EGFR inhibitor, a HER inhibitor (such as a HER-2 inhibitor), an anti-CD20 antibody molecule, a PARP inhibitor, a RON inhibitor, an anti-CD30 antibody, an anti-VEGF antibody, multispecific forms comprising any one of the same and conjugated forms of any one of the same.
22. A salt, composition, method or use according to any one of paragraphs 14 to 21, wherein the further therapeutic agent is a chemotherapeutic agent or combination thereof.
23. A salt, composition, method or use according to paragraph 22, wherein the chemotherapeutic agent is selected from the group comprising a platinum based chemotherapeutic, fluorouracil (5-FU), capecitabine, Raltitrexed, taxol and derivatives thereof, BGC 945, OSI-7904L, FOLFOX, FOLFIRI and FOLFIRINOX, and combinations of two or more of the same.
24. A salt, composition, method or use according to paragraph 23, wherein the platinum based chemotherapeutic agent is selected from the group comprising cisplatin, carboplatin, oxaliplatin, satraplatin, picoplatin, nedaplatin, triplatin, lipoplatin and combinations thereof, in particular cisplatin.
25. A salt, composition, method or use according to any one of paragraphs 14 to 24, wherein the further anticancer agent is a DHODH inhibitor.
26. A salt, composition, method or use according to paragraph 25, wherein the DHODH inhibitor is selected from: teriflunomide, leflunomide, brequinar, atovaquone; 2-(3,5-difluoro-3'-methoxybiphenyl-4-ylamino)nicotinic acid (or a pharmaceutically acceptable salt thereof, for example a salt disclosed in WO2010/102826, such as the meglumine salt) 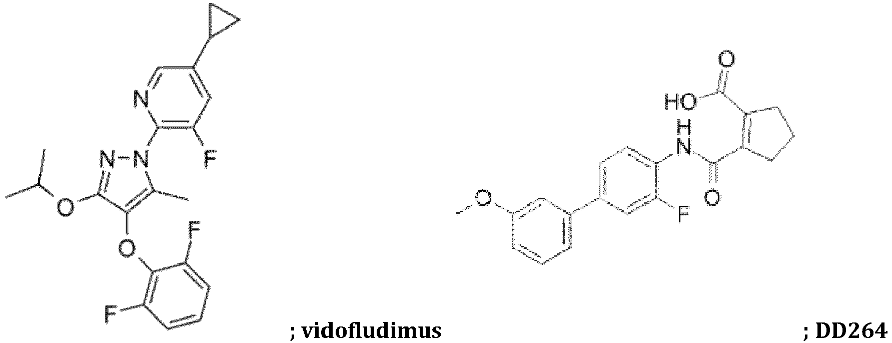 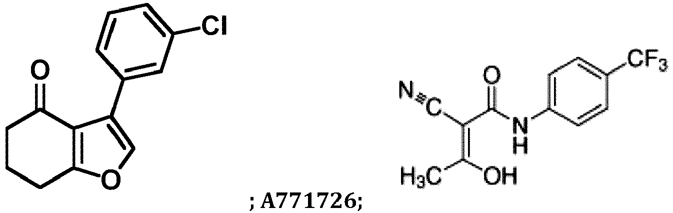 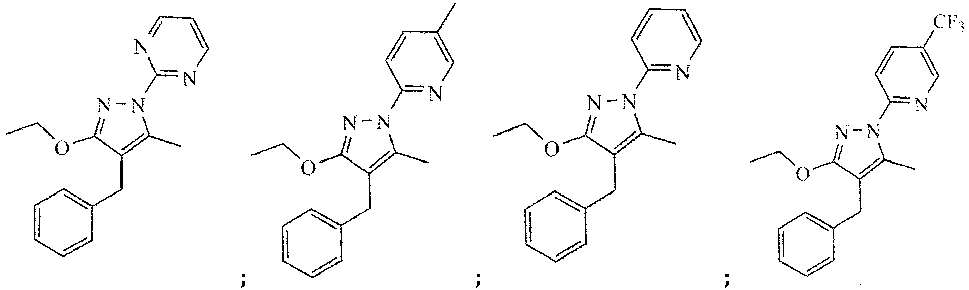 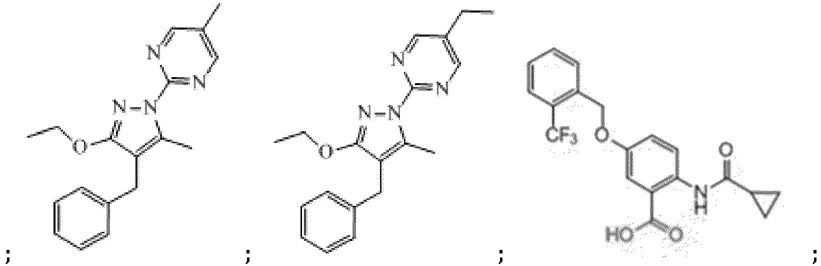 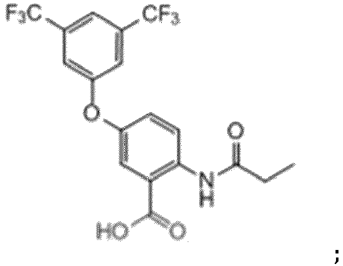 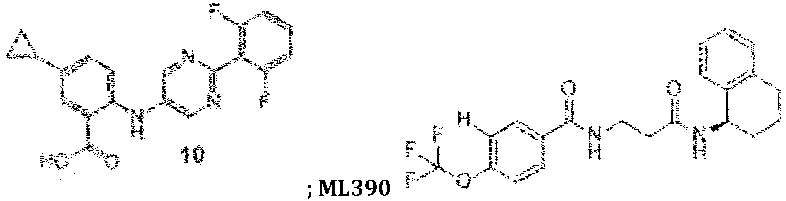 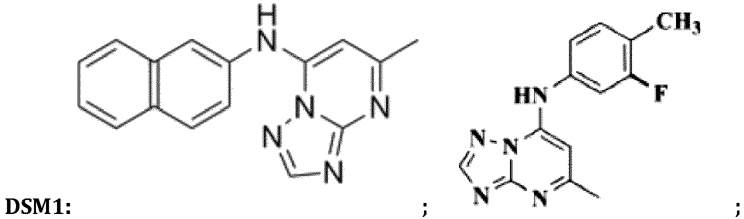 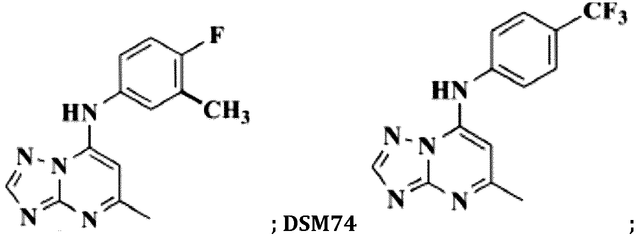 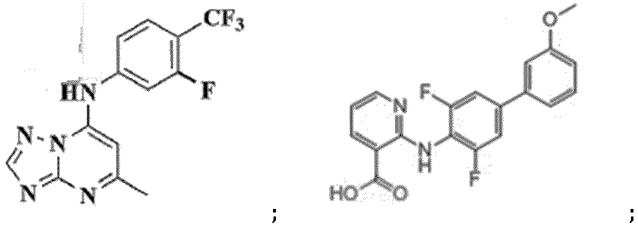 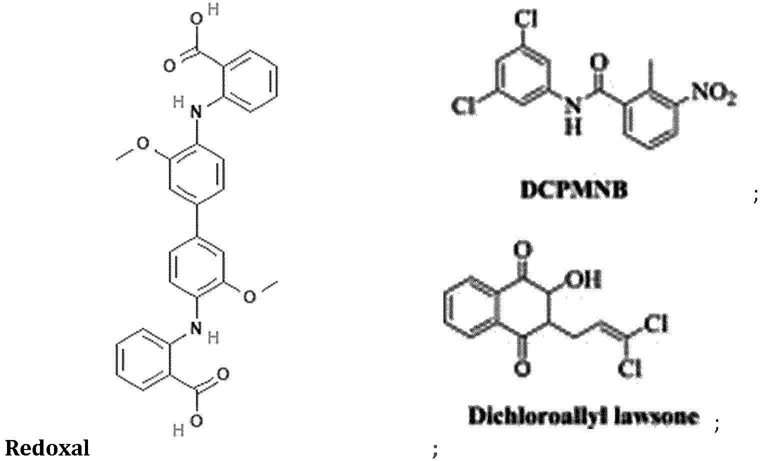 a compound of formula (IV): 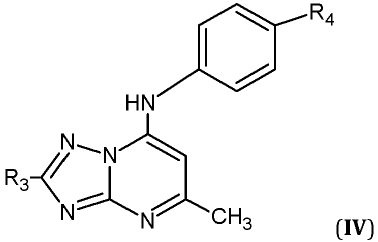 wherein
   R³ is CF₂CF₃, CF₃, OCH₂CH₃ or CH₂CH₃;
   R⁴ is F, CH₃, CF₃, SF₃ or CL, or
   a pharmaceutically acceptable salt of any one of the same.
27. A salt, composition or use according to any one of paragraphs 14 to 26, wherein the further therapeutic agent is a small molecule PARP inhibitor.
28. A salt, composition or use according to any one of paragraphs 14 to 27 for use in sensitizing a cancer patient to chemotherapy.
29. A salt, composition or use according to any one of paragraphs 14 to 28, wherein the cancer is selected from the group consisting of: liver cancer (such as hepatocellular carcinoma), biliary tract cancer, breast cancer (such as none ER+ breast cancer), prostate cancer, colorectal cancer, ovarian cancer, cervical cancer, lung cancer, gastric cancer, pancreatic, bone cancer, bladder cancer, head and neck cancer, thyroid cancer, skin cancer, renal cancer, and oesophagus cancer.
30. A salt, composition or use according to paragraph 29, wherein the cancer is selected from the group consisting of: gastric cancer, hepatocellular carcinoma and cholangiocarcinoma, for example cholangiocarcinoma.
31. A salt, composition or use according to any one of paragraphs 14 to 30, wherein the cancer is refractory and/or resistant to a least one cancer therapy, such as chemotherapy.
32. A salt, composition or use according to any one of paragraphs 14 to 33, wherein the cancer is HER positive (such as HER 1, HER 2, HER 3 and/or HER4 positive) or HER amplified (such as HER 1, HER 2, HER 3 and/or HER4 amplified).
33. An active pharmaceutical ingredient (API) of formula (**I**) as defined above or an enantiomer thereof provided as the free base, which comprises 0.1%, or less, such as 0.09 or less, 0.08% or less, 0.06% or less, 0.05% or less of an aniline impurity C₁₈H₁₄ClN₅OS, for example when analysed by HPLC or similar techniques and expressed as an area/area value (% area).
34. An API according to paragraph 33, wherein the compound is of formula (**IA**) defined above.
35. An API according to paragraphs 33 or 34, wherein the aniline impurity has the structure of formula (**II**) defined above.
36. An API according to any one of paragraphs 33 to 35, wherein the free base comprises 5 or less, for example 1, 2, 3, or 4 impurities above the limit of detection.
37. An API according to any one of paragraphs 33 to 36, wherein the free base contains thiazoline impurity C₂₂H₁₉ClN₆OS₂ at a level of 1.5% or less, such as 1.5%, 1.4%, 1.3%, 1.2%, 1.1%, 1%, 0.9%, 0.8%, 0.7%, 0.6%, 0.5% or less, for example when analysed by HPLC or similar techniques and expressed as an area/area value.
38. An API according to paragraph 37, wherein the thiazoline impurity has the structure of formula (**III**) defined above.
39. An API according to paragraphs 37 or 38, wherein said thiazoline is present at 0.75% or less, such as 0.5% or less, in particular 0.4, 0.3, 0.2, 0.1% or less.
40. An API according to any one of paragraphs 33 to 39, which has a purity of least 97%, for example 97.5%, 97.6, 97. 7, 97.8, 97.9, 98.0, 98.1, 98.2, 98.3, 98.4, 98.5, 98.6, 98.7, 98.8, 98.9, 99.0, 99.1, 99.2, 99.3, 99.4, 99.5% or above.
41. A pharmaceutical composition comprising the API according to any one of paragraphs 33 to 40.
42. A pharmaceutical composition according to claim 41, provided as one or more unit doses, for example one or more tablets or capsules.
43. A pharmaceutical composition according to paragraph 41 or 42, wherein the composition is for oral administration.
44. A pharmaceutical composition according to any one of paragraphs 41 to 43, wherein the dose of free base is in the range 100mg to 900mg, such as 200mg to 500mg, in particular 200mg, 300mg, or 400mg.
45. An API according to any one of paragraphs 33 to 39 or a pharmaceutical composition according to any one of paragraphs 41 to 44 for use in treatment.
46. An API or pharmaceutical composition according to paragraph 45 for use in treating cancer in particular a cancer disclosed herein, for example sensitizing a cancer patient to chemotherapy.
47. Use of an API according to any one of paragraphs 33 to 40 or a composition according to any one of paragraphs 41 to 44, for the manufacture of a medicament for the treatment of cancer.
48. An API, composition or use according to any one of paragraphs 33 to 47, wherein the API of formula (**I**) is administered bi-daily.
49. An API, composition or use according to any one of paragraphs 33 to 48, wherein the therapy comprises a further therapeutic agent
50. An API, composition or use according to paragraph 49, wherein the further therapeutic agent is an anticancer agent.
51. An API, composition or use according to paragraph 49 or 50, wherein the anticancer agent is a biological therapeutic agent, such as an antibody molecule.
52. An API, composition or use according to paragraph 51, wherein the biological therapeutic agent is an antibody molecule selected from the group comprising a PD-1 inhibitor, a PD-L1 inhibitor, a CTLA-4 inhibitor, EGFR inhibitor, a HER inhibitor (such as a HER-2 inhibitor), an anti-CD20 antibody molecule, a PARP inhibitor, a RON inhibitor, an anti-CD30 antibody, an anti-VEGF antibody, multispecific forms comprising any one of the same and conjugated forms of any one of the same.
53. An API, composition or use according to any one of paragraphs 49 to 52, wherein the further therapeutic agent is a chemotherapeutic agent or combination thereof, for example selected from a platinum based chemotherapeutic, a pyrimidine analogue and combinations of two or more of the same.
54. An API, composition or use according to paragraph 53, wherein the chemotherapeutic agent is selected from the group comprising a platinum based chemotherapeutic, fluorouracil (5-FU), capecitabine, Raltitrexed, taxol and derivatives thereof, BGC 945, OSI-7904L, FOLFOX, FOLFIRI and FOLFIRINOX, and combinations of two or more of the same.
55. An API, composition or use according to paragraph 54, wherein the platinum based chemotherapeutic agent is selected from the group comprising cisplatin, carboplatin, oxaliplatin, satraplatin, picoplatin, nedaplatin, triplatin, lipoplatin and combinations thereof, in particular cisplatin.
56. An API, composition or use according to any one of paragraphs 49 to 55, wherein the further anticancer agent is a DHODH inhibitor.
57. An API, composition or use according to paragraph 56, wherein the DHODH inhibitor is selected from: the list given in paragraph 26 above.
58. An API, composition or use according to any one of paragraphs 49 to 57, wherein the further therapeutic agent is a small molecule PARP inhibitor.
59. An API, composition or use according to any one of paragraphs 33 to 58, for use in sensitizing a cancer patient to chemotherapy.
60. An API, composition or use according to any one of paragraphs 33 to 59, wherein the cancer is selected from the group consisting of: liver cancer (such as hepatocellular carcinoma), biliary tract cancer, breast cancer (such as none ER+ breast cancer), prostate cancer, colorectal cancer, ovarian cancer, cervical cancer, lung cancer, gastric cancer, pancreatic, bone cancer, bladder cancer, head and neck cancer, thyroid cancer, skin cancer, renal cancer, and oesophagus cancer.
61. An API, composition or use according to paragraph 60, wherein the cancer is selected from the group consisting of: gastric cancer, hepatocellular carcinoma and cholangiocarcinoma, for example cholangiocarcinoma.
62. An API, composition or use according to any one of paragraphs 33 to 61, wherein the cancer is refractory and/or resistant to a least one cancer therapy, such as chemotherapy.
63. An API, compositionhod or use according to any one of paragraphs 33 to 62, wherein the cancer is HER positive (such as HER 1, HER 2, HER 3 and/or HER4 positive) or HER amplified

In one aspect, there is provided a malonate salt, composition or use according to the present disclosure for use in sensitizing a cancer patient to chemotherapy.

In one embodiment, the cancer is HER positive (such as HER 1 positive, HER 2 positive, HER 3 positive, HER 4 positive, HER 1 & HER 2 positive, HER 1 & HER 3 positive, HER 1 & HER 4 positive, HER 2 & HER 3 positive, HER 2 & HER 4 positive, HER 1, HER 2 & HER 3 positive; HER 1, HER 2 & HER 4 positive; HER 2, HER 3 & HER4 positive; HER 1, HER 2, HER 3 & HER4 positive) or HER amplified (such as HER 1, &/or HER 2 &/or HER 3 &/or HER4 amplified).

Also provided as an independent aspect are novel polymorphic forms disclosed herein, pharmaceutical formulations compositions comprising the same and use of any one of the same in treatment, in particular the treatment of cancer, such as a cancer disclosed herein.

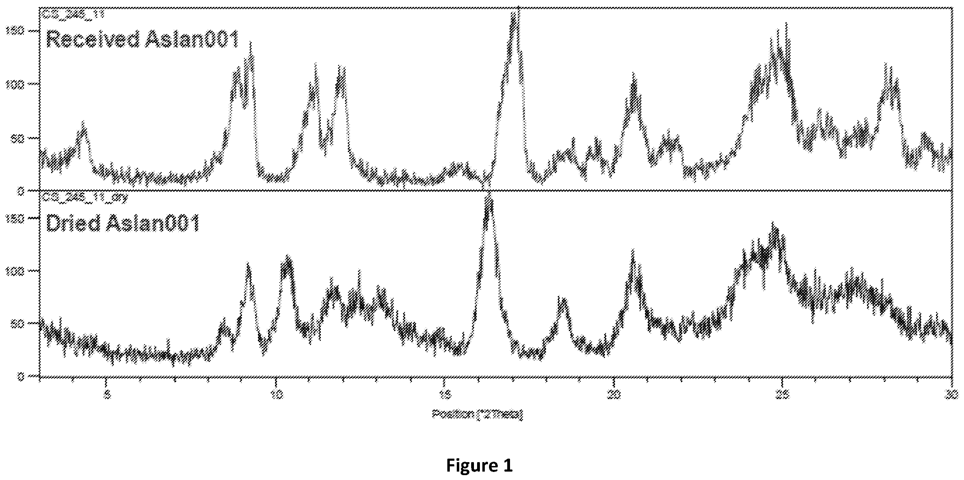

In one aspect the polymorphic form is the anhydrous alpha form shown in Figure 1, for example (R)-N4-[3-Chloro-4-(thiazol-2-ylmethoxy)-phenyl]-N6-(4-methyl-4,5,-dihydro-oxazol-2-yl)-quinazoline-4,6-diamine (varlitinib) exhibiting at least X-ray lines (2-theta values) in a powder defraction pattern (in particular when measured using Cu Kalpha radiation) at 9.2 +/-0.2, 10.35 +/-0.2,16.25 +/- 0.2, 18.6 +/-0.2, 18.6 +/-0.2 and optionally 20.0 +/-0.2.

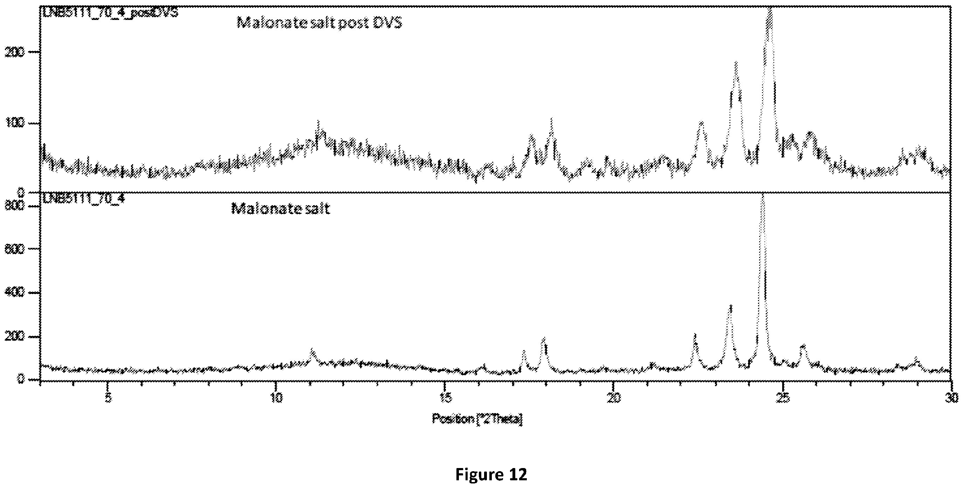

In one embodiment the polymorphic form is shown in Figure 12.

In one aspect, there is provided a process for manufacturing a salt as described above comprising mixing a compound of formula (**I**) or an enantiomer thereof (such as varlitinib) in the free base form with malonic acid in the presence of a suitable solvent or mixture of two or more solvents.

In one embodiment the crude stage material (such as crude stage 7 material) is employed to make the salt according to the present disclosure. This is advantageous because it removes two steps of the present manufacturing process.

In one embodiment the stage 9 free base is employed to make a salt according to the present disclosure.

In one embodiment the process further comprises drying a compound of formula (**I**) or an enantiomer thereof (such as varlitinib) prior to dissolving the compound of formula (**I**) or an enantiomer thereof in the solvent

In one embodiment, the method further comprises isolating the salt, for example by a dissolution-crystallisation-cooling process

In one embodiment the salt is isolated by temperature cycling in the range 1 to 40 °C in 4 hour cycles.

In one embodiment the temperature cycling is carried out for 24 hours or more.

In one embodiment the cooling/heating rates are 1 °C/min.

In one embodiment the solvent employed in the process is selected from the group comprising water, methanol, ethanol, isopropyl alcohol, n-propanol, tert-butanol, n-butanol, methylene chloride, ethyl dichloride, chloroform, carbon tetrachloride, acetone, methyl isobutyl ketone, N, N-dimethylformamide, N,N-dimethylacetamide, dimethylsulfoxide, acetonitrile, propionitrile, ethyl acetate, isopropyl acetate, acetone, diisopropyl ether, methanol, tetrahydrofuran (THF) and combinations thereof.

In one embodiment the solvent employed in the process is selected from the group consisting of: water, methanol, ethanol, isopropyl alcohol, n-propanol, tert-butanol, n-butanol, methylene chloride, ethyl dichloride, chloroform, carbon tetrachloride, acetone, methyl isobutyl ketone, N,N-dimethylformamide, N,N-dimethylacetamide, dimethylsulfoxide, acetonitrile, propionitrile, ethyl acetate, isopropyl acetate, acetone, diisopropyl ether, tetrahydrofuran (THF) and acetonitrile: water, for example acetone, diisopropyl ether, tetrahydrofuran (THF) or acetonitrile:water.

In one embodiment the solvent is selected from the group consisting of acetone, diisopropyl ether, ethyl acetate, methanol, tetrahydrofuran (THF) and acetonitrile:water, such as THF.

In one embodiment the solvent is a combination of THF and water.

In one embodiment the solvent is a combination of acetone and water.

In one embodiment the solvent is acetone.

In one embodiment the solvent is a polar aprotic solvent, for example tetrahydrofuran, in particular the solvent is employed in an anhydrous form.

In one embodiment, the compound of formula (**I**) or the enantiomer thereof is dissolved in 10-15 volumes of solvent for example 12 volumes of the solvent, expressed as litres per Kg (for example 1Kg of solid requires at 10 Litres of solvent).

In one embodiment the amount of malonic acid employed is equivalent in mass to the amount (in for example g or Kgs) of solvent employed.

In one embodiment the malonic acid as one or more molar equivalents is added to the free base, for example 1, 1.1, 1.25, 1.5, 1.75, 2, 2.25, 2.5, 2.75 or 3 molar equivalents.

In one embodiment, the process further comprises a step of converting the malonate salt into the free base, for example by dissolving the salt in at least one solvent followed by adding a base.

In one embodiment the process comprises formulating a malonate salt or free base derived therefrom as a pharmaceutical composition.

In one embodiment the free base according to the present disclosure or a salt is provided as a pharmaceutical composition.

In one aspect, there is provided a malonate salt, a free base or a pharmaceutically acceptable salt obtainable by a method according to the present disclosure.

The present inventors have surprisingly found that by converting the free base into a malonate salt and then converting the malonate salt back into the free base, particular impurities can be reduced in a way that is not possible going through salts, such as the ditosylate salt. This allows a pharmaceutically active ingredient (API) in the form of the malonate salt or the free base derived therefrom or a salt derived from the latter to be prepared on a commercial scale (as opposed to the laboratory scale) with a desirable impurity profile.

Hence, in one aspect, there is provided a pharmaceutical active ingredient (API) of formula (**I**) defined above or an enantiomer thereof provided a free base, which comprises 0.1%, 0.09%, 0.08%, 0.06%, 0.05%, or less of an aniline impurity, such as C₁₈H₁₄ClN₅OS.

In one embodiment the API (the free base or salt at the last stages of manufacturing) has not been subject to preparative liquid chromatography, such as HPLC.

Advantageously, when the API is prepared on a commercial scale, it has an improved purity profile. Removing impurities may also contribute to an improved stability profile.

In one embodiment, the compound has a formula (**IA**) defined above.

In one embodiment, the free base contains a thiazoline impurity with a molecular mass of about 483.

In one embodiment, the thiazoline impurity is C₂₂H₁₉ClN₆OS₂.

In one embodiment, the thiazoline impurity has the structure of formula (III) defined above.

In one embodiment, said thiazoline impurity is present at 1.5% w/w or less, for example 1% w/w or less, such as 0.9% w/w or less, 0.8% w/w or less, or 0.7% w/w or less, in particular 0.6% w/w or less.

In one embodiment, said thiazoline is present at 0.75% w/w or less, such as 0.5% w/w or less, in particular 0.4, 0.3, 0.2, 0.1% w/w or less.

In one embodiment, the API has a purity of least 97% w/w, for example 97.5%, 97.6, 97. 7, 97.8, 97.9, 98.0, 98.1, 98.2, 98.3, 98.4, 98.5, 98.6, 98.7, 98.8, 98.9, 99.0, 99.1, 99.2, 99.3, 99.4, 99.5% w/w or above.

In one aspect, there is provided a pharmaceutical composition comprising the API as herein or a salt derived from same.

In one embodiment, the pharmaceutical composition is provided as one or more unit doses.

In one embodiment, the pharmaceutical composition is for oral administration.

In one embodiment, the dose of free base is in the range 100mg to 900mg, such as 200mg to 500mg, in particular 200mg, 300mg, or 400mg.

In one aspect, there is provided an API, a salt derived therefrom or a pharmaceutical composition comprising any one of the same as described herein for use in treatment, for example for use in treating cancer, such as for use in sensitizing a cancer patient to chemotherapy.

The disclosure provides a method of treatment comprising administering a therapeutically effective amount of an API or a composition as described above.

In one embodiment, there is provided an API, a salt derived thereof or a pharmaceutical composition comprising any one of the same as for use in a method of treatment, in particular for the treatment of cancer, such as for use in sensitizing a cancer patient to chemotherapy comprising administering a therapeutically effect amount to a patient in need thereof.

In one aspect, there is provided the use of an API, salt derived therefrom or a composition comprising any one of the same as described herein for the manufacture of a medicament for the treatment of cancer.

In one embodiment, the API, salt derived therefrom or a composition comprising any one of the same is administered bi-daily.

In one embodiment, the API therapy comprises a further therapeutic agent

The therapies and uses described herein in respect of compounds of formula (**I**) apply equally the API of the present disclosure.

In one aspect, there is provided a method of producing a free base of a compound of formula (**I**) or an enantiomer thereof, comprising dissolving a salt as described above in at least one solvent followed by adding a base.

In one embodiment the solvent employed in the process is selected from the group comprising water, methanol, ethanol, isopropyl alcohol, n-propanol, tert-butanol, n-butanol, methylene chloride, ethyl dichloride, chloroform, carbon tetrachloride, acetone, methyl isobutyl ketone, N,N-dimethylformamide, N,N-dimethylacetamide, dimethylsulfoxide, acetonitrile, propionitrile, ethyl acetate, isopropyl acetate, acetone, diisopropyl ether, methanol, tetrahydrofuran (THF) and combinations thereof.

In one embodiment the solvent is selected from the group consisting of: water, methanol, ethanol, isopropyl alcohol, n-propanol, tert-butanol, n-butanol, methylene chloride, ethyl dichloride, chloroform, carbon tetrachloride, acetone, methyl isobutyl ketone, N,N-dimethylformamide, N,N-dimethylacetamide, dimethylsulfoxide, acetonitrile, propionitrile, ethyl acetate, isopropyl acetate, acetone, diisopropyl ether, ethyl acetate, methanol, tetrahydrofuran (THF) and acetonitrile:water, for example acetone, diisopropyl ether, tetrahydrofuran (THF) or acetonitrile: water.

In one embodiment the solvent is a polar aprotic solvent, for example tetrahydrofuran, in particular the solvent employed in anhydrous.

In one embodiment the solvent is a combination of THF, water and ethanol.

In one embodiment the base is selected from the group comprising an alkali metal carbonic acid salt such as lithium carbonate, sodium carbonate, potassium carbonate and cesium carbonate; an alkali metal hydrogen carbonic acid salt such as lithium hydrogen carbonate, sodium hydrogen carbonate and potassium hydrogen carbonate; an alkali metal hydride such as lithium hydride, sodium hydride and potassium hydride; an alkali metal hydroxide such as lithium hydroxide, sodium hydroxide and potassium hydroxide; an alkali metal alkoxide such as lithium methoxide, sodium methoxide, sodium ethoxide and potassium t-butoxide; or an organic amine such as triethylamine, tributylamine, 4-(N,N-dimethylamino)pyridine, N,N-diisopropylethylamine, N-methylmorpholine, pyridine, N,N-dimethylaniline, N,N-diethylaniline, 1,5-diazabicyclo-[4.3.0]non-5-ene, 1,4-diazabicyclo[2.2.2]octane (DABCO) and 1,8-diazabicyclo[5.4.0]-7-undecene (DBU), and it is preferably 4-(N,N-dimethylamino)pyridine, N,N-diisopropylethylamine, alkali metal hydride or an alkali metal carbonic acid salt and most preferably 4-(N,N-dimethylamino)pyridine, N,N-diisopropylethylamine, sodium hydride, potassium carbonate and cesium carbonate.

In one aspect, there is provided a free base obtainable by a method as described above.

In one embodiment the malonate salt or free base (including API) of compound of formula (I) such as varlitinib, a salt derived from the same or a pharmaceutical composition thereof according to the present disclosure, is employed in a combination therapy, for example with a further HER inhibitor, for example a combination of a compound of formula (**I**) (such as Varlitinib) and Herceptin (trastuzumab) and/or pertuzumab. Surprisingly a combination of Varlitinib and Herceptin show more therapeutic activity than either entity alone.

In one embodiment, the combination comprises ado-trastuzumab-emtansine and a malonate salt, free base (including API) of compound of formula (**I**) such as varlitinib or a pharmaceutically acceptable salt derived therefrom or pharmaceutical composition thereof comprising any one of the same.

These combinations of HER inhibitors may be employed with a chemotherapy agent, for example a chemotherapeutic agent disclose herein.

Varlitinib at an appropriate dose is capable of inhibiting HER1, HER2 and HER4 directly and thought to be capable of inhibiting HER3 indirectly.

In one embodiment, the malonate salt, or free base (including API) of compound of formula (**I**), such as Varlitinib, a pharmaceutically acceptable salt derived therefrom or a pharmaceutical composition of any one of the same at least inhibits the activity of HER1 and HER2, HER1 and HER4 or HER2 and HER4.

In one embodiment, the salt or free base(including API) of compound of formula (**I**), such as varlitinib, or pharmaceutical composition thereof at least inhibits the activity of HER1, HER2 and/or HER4, for example directly inhibits the activity of HER1, HER2 and/or HER4.

In one embodiment, the salt or free base (including API) of compound of formula (**I**), such as varlitinib, or pharmaceutical composition thereof inhibits the activity of HER1, HER2, HER3 and HER4, for example directly inhibits the activity of HER1, HER2, and HER4, and indirectly inhibits the activity of HER3.

In one embodiment the malonate salt, free base (including API) of the compound of formula (I), such as varlitinib, pharmaceutically acceptable salt derived therefrom or pharmaceutical composition thereof according to the present disclosure is administered in a 28 days treatment cycle.

In one embodiment the target patient population is EGFR and HER2 positive or are HER2 amplified.

In one embodiment the treatment of the present disclosure is administered for a non-epithelial cancer in which HER inhibition is effective.

In one embodiment the treatment of the present disclosure is administered for epithelial cancer, for example as listed in paragraph 31, for example gastric cancer. In one embodiment the epithelial cancer is a carcinoma.

In one embodiment the cancer is selected from selected from the group comprising hepatocellular carcinoma, cholangiocarcinoma, breast cancer, prostate cancer, colorecetal cancer, ovarian cancer, lung cancer, gastric cancer, pancreatic and oesophagus cancer.

In one embodiment the biliary duct cancer is in a location selected from intrahepatic bile ducts, left hepatic duct, right hepatic duct, common hepatic duct, cystic duct, common bile duct, Ampulla of Vater and combinations thereof.

In one embodiment the biliary duct cancer is in an intrahepatic bile duct. In one embodiment the biliary duct cancer is in a left hepatic duct. In one embodiment the biliary duct cancer is in a right hepatic duct. In one embodiment the biliary duct cancer is in a common hepatic duct In one embodiment the biliary duct cancer is in a cystic duct. In one embodiment the biliary duct cancer is in a common bile duct. In one embodiment the biliary duct cancer is in an Ampulla of Vater.

In one embodiment the therapy, such as combination therapy according to the disclosure is adjuvant therapy, for example after surgery.

In one embodiment the therapy, such as combination therapy according to the disclosure is neoadjuvant treatment, for example to shrink a tumour before surgery.

In one embodiment the tumour is a solid tumour. In one embodiment the cancer is a primary cancer, secondary cancer, metastasis or combination thereof. In one embodiment the treatment according to the present disclosure is suitable for the treatment of secondary tumours. In one embodiment the cancer is metastatic cancer. In one embodiment the treatment according to the present disclosure is suitable for the treatment of primary cancer and metastases. In one embodiment the treatment according to the present disclosure is suitable for the treatment of secondary cancer and metastases. In one embodiment the treatment according to the present disclosure is suitable for the treatment of primary cancer, secondary cancer and metastases.

In one embodiment the treatment according to the present disclosure is suitable for the treatment of cancerous cells in a lymph node, for a cancer of the present disclosure.

In one embodiment the liver cancer is primary liver cancer. In one embodiment the liver cancer is secondary liver cancer. In one embodiment the liver cancer is stage 1, 2, 3A, 3B, 3C, 4A or 4B.

In one embodiment the gastric cancer is stage 0, I, II, III or IV.

In one embodiment varlitinib is continued as a monotherapy, for example after the combination therapy of the present disclosure, for example administered once or twice daily at a dose in the range 100mg to 500mg, such as 200mg, 300mg or 400mg.

In one embodiment the monotherapy is administered for 1, 2, 3, 4, 5, 6, 7, 8, 9,10,11,12,13, 14,15,16,17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 months or more.

In one embodiment the patient is a human.

### DETAILED DISCLOSURE

References to compounds of formula (I) herein include compounds of formula (IA) unless indicated otherwise.

"Pharmaceutically acceptable salt" as employed herein refers to a salt form that is suitable for use in therapy, in particular human therapy.

Examples of pharmaceutically acceptable salts include but are not limited to an alkali metal salt such as a sodium salt, a potassium salt and a lithium salt; an alkali earth metal salt such as a calcium salt and a magnesium salt; a metal salt such as an aluminum salt, an iron salt, a zinc salt, a copper salt, a nickel salt, a cobalt salt and so on; an amine salt such as an ammonium salt, a t-octylamine salt, a dibenzylamine salt, a morpholine salt, a glucosamine salt, a phenyl glycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-N-phenethylamine salt, a piperazine salt, a tetramethylammonium salt and a tris(hydroxymethyl)-aminomethane salt; an amino acid salt such as a lysine salt, an ornithine salt and an arginine salt; a halide acid salt such as a hydrofluoric acid salt, a hydrochloric acid salt, a hydrobromic acid salt and a hydroiodic acid salt; a nitric acid salt; a perchloric acid salt; a sulfuric acid salt; a phosphoric acid salt; a sulfonic acid salt such as a methanesulfonic acid salt, a trifluoromethanesulfonic acid salt, an ethanesulfonic acid salt, a benzenesulfonic acid salt and a p-toluenesulfonic acid salt; a carboxylic acid salt such as an acetic acid salt, a malic acid salt, a fumaric acid salt, a succinic acid salt, a citric acid salt, a tartaric acid salt, an oxalic acid salt and a maleic acid salt; or an amino acid salt such as a glutamic acid salt and an aspartic acid salt, and it is preferably an alkali metal salt, an alkali earth metal salt, a amine salt, a halide acid salt, a nitric acid salt, a sulfuric acid salt, a phosphoric acid salt, a sulfonic acid salt, a carboxylic acid salt or an amino acid salt, and more preferably a sodium salt, a potassium salt, a lithium salt, a calcium salt, a magnesium salt, an ammonium salt, a morpholine salt, a glucosamine salt, a phenylglycine alkyl ester salt, an ethylenediamine salt, an N-methylglucamine salt, a guanidine salt, a chloroprocaine salt, a procaine salt, a tetramethylammonium salt, a lysine salt, a hydrochloric acid salt, a hydrobromic acid salt, a nitric acid salt, a sulfuric acid salt, a phosphoric acid salt, a methanesulfonic acid salt, a p-toluenesulfonic acid salt, an acetic acid salt, a malic acid salt, a fumaric acid salt, a succinic acid salt, a citric acid salt, a tartaric acid salt, an oxalic acid salt, a maleic acid salt, an ornithine acid salt, a glutamic acid salt or an aspartic acid salt.

In one embodiment, the salt is produced from an acid selected from the group consisting of: adipic acid, benzene sulphonic acid, benzoic acid, citric acid, 2-5-dihydroxybenzoic acid, ethane disulphonic acid, fumaric acid, galataric acid, 1-hydroxy-2-napthoic acid, maleic acid, malic acid, malonic acid, 1,5-napthelene disulphonic acid, oxalic acid, succinic acid and DL-tartaric acid, for example adipic acid, galataric acid, maleic acid or malonic acid. Thus, in one embodiment the acid is malonic acid.

In one embodiment the pharmaceutically acceptable salts include but are not limited to acid addition salts of strong mineral acids such as HCl and HBr salts and addition salts of strong organic acids, such as a methansulfonic acid salt, tosylates, furoates and the like, including di, tri salts thereof, such as ditosylates.

Pharmaceutically acceptable salts that can be used include mineral acid salts, such as hydrochlorides, hydrobromides, phosphates and sulphates, or salts of organic acids, such as acetates, propionates, malonates and benzoates.

"Malonate salt" as used herein generally refers to an acid addition salt formed from the addition of malonic acid. Thus, malonate salt in the context of the present disclosure refers to a compound of formula (**I**), in particular varlitinib, where there is a counter-ion from malonic acid.

An enantiomer thereof as employed herein refers to wherein one stereoisomer is provided in excess of other stereoisomers, for wherein one stereoisomer is provided in at least 70% enantiomeric excess (ee), such as 75, 80, 85, 90, 91, 92, 93, 94, 95, 96, 97, 98,99 or 100% ee.

The free base in the context of the present disclosure refers to the compound of formula (**I**), such as Varlitinib, for example without a counter-ion, for example in the form of a deprotonated amine. The free base is really only available in solution. When the free base is in a solid physical form a hydrogen counter-ion will associated with the base, such that the overall charge of the molecule is zero. The term free base is generally used in the art to include non-dissolved forms of the molecule without a counterion. The present specification uses the term free base in the same way as generally employed in the art and thus includes all physical forms of the molecule.

Unless the context indicated otherwise "salt derived therefrom" as employed herein refers to a salt made from a free base of formula (**I**), in particular varlitinib, which was liberated from a malonate salt of the present disclosure and therefor has the higher levels of purity as discussed herein. A pharmaceutically acceptable salt made from that free base is purer than previously possible and hence has the novel and beneficial properties of the free base it was made from.

A pharmaceutically acceptable ingredient (API) as employed herein refers to an active pharmaceutical agent, therapeutic, made on a commercial scale, for example in a manufacturing plant as opposed to a laboratory. Different equipment and processes are used on a plant scale, which is some instances limits the synthetic routes that are available to make the compound. An API will usually be made on a scale of at least 0.5Kg in one batch, for example 1, 2, 3, 4, 5, 6, 7, 8, 9, 10, 11, 12, 13,14,15,16,17,18,19, 20, 21, 22, 23, 24 or 25 Kgs. On the laboratory scale usually several batches need to be combined to make 0.5Kgs. In one embodiment, the API is made on a scale of 50 kg or more, such as 55, 60, 65, 70, 75, 80, 85, 90, 95, 100 kg.

Material made at a small scale in the laboratory will usually be referred to as a "compound". In contrast, "API" as employed herein is a term used to refer to compound made by a commercial manufacturing route, which is employed to supply active material for clinical trials or for commercial sales.

API as employed herein does not include preparation on the laboratory scale.

API are not purified by high performance liquid chromatography, such as HPLC or LCMS.

Active pharmaceutical ingredient or agent as employed herein refers to the compound which provides a pharmacological effect, in particular an efficacious pharmacological effect.

The conversion of a compound of formula (**I**) into a pharmaceutically acceptable salt may be effected in conventional manner, for example, by treatment of the compound with an appropriate acid to form an acid addition salt This may be carried out for example by dissolving the compound in an appropriate solvent or mixture of solvents before adding a suitable acid. Optionally, the compound of formula (**I**) or enantiomer thereof is dried in order to remove water or residual solvents before it is dissolved in the solvent.

Thus, the present disclosure provides a method of manufacturing a pharmaceutically acceptable salt comprising dissolving a compound of formula (**I**) or an enantiomer thereof in at least one solvent followed by, for example adding malonic acid.

The salt produced may then be isolated by using any appropriate method and drying equipment common to large scale API manufacture, which are known to the skilled addressee. For example, by drying the salt using temperature cycling. A vacuum tray dryer, a rotocon vacuum Dryer, a vacuum paddle dryer or a pilot plant rota vapor, may be used to further lower residual solvents.

In one embodiment, the drying is carried out at atmospheric pressure or reduced pressures, such as below about 200 mm Hg, or below about 50 mm Hg, at temperatures such as about 35° C. to about 70° C. The drying can be carried out for any desired time period that achieves the required result, such as about 1 to 20 hours. Drying may also be carried out for shorter or longer periods of time depending on the product specifications. Temperatures and pressures will be chosen based on the volatility of the solvent being used and the foregoing should be considered as only a general guidance. Drying may be suitably carried out in a tray dryer, vacuum oven, air oven, or using a fluidized bed drier, spin flash dryer, flash dryer and the like. Drying equipment selection is well within the ordinary skill in the art

The skilled person will be aware of other methods for producing salts.

"High purity" as used herein refers to having a purity of at least 95%, such as 95.5%, 96.0%, 96.5%, 97%, 97.5%, 98%, 98.5%, 99%, 99.5%, or 100%.

In one embodiment the salts of the present disclosure, in particular the malonate salt, have suitable stability.

This may be because reducing the level of impurities may remove sources and routes of certain synthetic degradation.

The following are impurities that may be present in the final form of compounds of formula (**I**), such as varlitinib:
Carbamate: 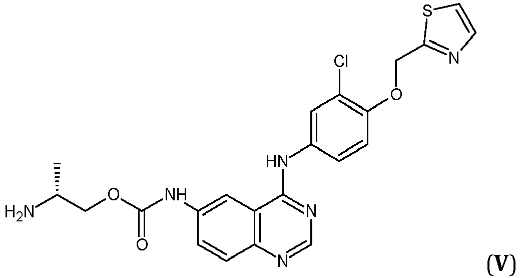
Hydroxyurea

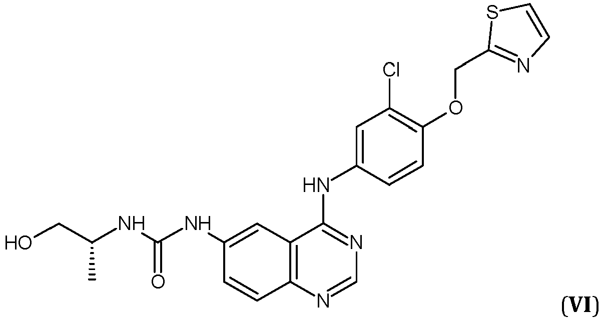

Aniline a compound formula (**II**) disclosed above; and Thiazoline (a compound of formula (**III**) disclosed above).

"Suitable stability" as used herein refers to a salt that does not exhibit any significant signs of change when stored for at least 1 week, for example 2, 3, 4, 5, 6, 7, 8, 9, 10, 11 or 12 weeks. In one embodiment, the malonate salt (or the free base derived therefrom) does not exhibit any significant signs of change when stored for at least 1 week at 40 °C/75% RH. In one embodiment, the malonate salt (or the free base derived therefrom) does not exhibit any significant signs of change when stored for at least 1 week and exposed to ambient light In one embodiment, the malonate salt (free base or salt derived therefrom) does not exhibit any significant signs of change when stored for at least one 1 week at 80 °C. In one embodiment high stability refers to the ability to store the malonate salt, API free base or a pharmaceutically acceptable salt derived therefrom for at least 12 months such as 18 months, in particular 2 years, especially at ambient temperature.

Conversion of a malonate salt of a compound of formula (**I**) or enantiomer thereof, in particular varlitinib, into the free base of compound of formula (**I**) or enantiomer thereof, may be effected in conventional manner, for example by dissolving the salt in a solvent, followed by adding a base to convert a salt of the present disclosure into a free base of compound formula (**I**).

In one aspect, there is provided a method of producing a free base of a compound of formula (**I**) or an enantiomer thereof comprising dissolving a salt of a compound of formula (**I**) or an enantiomer thereof in a solvent followed by adding a base.

In one embodiment, the free base may be prepared by dissolving an acid addition salt of a pharmaceutical in a basic solution (e.g. sodium hydroxide in an organic/aqueous co-solvent). Additional base may be added to ensure that the acid addition salt is substantially converted to the free base.

In one embodiment the free base is prepared by dissolving a salt of a compound of formula (**I**) or an enantiomer thereof in a mixture of THF, ethanaol and water.

In another embodiment, water and acetone are combined to form an aqueous-organic co-solvent, which may then be used to dissolve the salt. The co-solvent system is then basified using a weak base, e.g., by adding a solution of an organic or inorganic basic compound to the co-solvent system. While the base is being added, the acid addition salt undergoes conversion to the free base.

In one embodiment, the organic free base is completely dissolved by the organic solvent and the water dissolves the functional acid and the resulting solution contains a completely dissolved organic free base in an aqueous-organic solvent.

In one embodiment, water in the solvent system allows for processing temperatures to be raised higher than if the solvent were strictly organic.

Varlitinib has significant anticancer activity when employed as a monotherapy. However, increased efficacy is observed with varlitinib is employed in combination therapy, in particular with chemotherapy, even when the patient did not respond to chemotherapy in the absence of varlitinib. Whilst not wishing to be bound by theory the administration of varlitinib seems to restore at least some of the therapeutic activity of the chemotherapy, in resistant or refractory patients.

In one embodiment the compound of formula (I) according to the present disclosure sensitize patient to platin and/or a pyrimidine analogue.

In one embodiment the chemo-sensitization is via inhibition of MATE 1 and/or MATE 2.

In one embodiment the chemo-sensitization is via inhibition of ABCG2.

"Sensitization to chemotherapy" as employed herein simply refers to the fact a combination of the compound of formula (**I**) with a chemotherapy has increased efficacy in comparison to one or both therapies employed alone. The sensitization treatment according to the present disclosure can be employed in patients who have primarily resistant to treatment, such as chemotherapy. The sensitization treatment according to the present disclosure can be employed in patients who have secondary resistant to treatment, such as chemotherapy. The sensitization treatment according to the present disclosure can be employed in patients who have not shown resistance, in an attempt to avoid them becoming resistant

Thus in one embodiment sensitizing a cancer patient to chemotherapy as employed herein refers to rendering the cancer cells more susceptible to the therapeutic/killing effects of chemotherapy and/or to the compound of formula (**I**) such as varlitinib, or reciprocally to both therapies, in comparison to the "same" cancer cells without treatment with a compound of formula (I).

To obtain the benefits of sensitization to chemotherapy then the chemotherapy has to administered in a time frame, where the pharmacological effects of a compound of formula (**I**) and the chemotherapy overlap, i.e. the treatment regimens for the said therapies partly coincide in time A skilled person will understand in practice what this means.

"Administering a combination therapy" as employed herein does not require the therapies employed in the combination to be administered at the same time.

Combination therapy as employed herein refers to two or more modes of therapy being employing over the same treatment period, i.e. the opposite of sequential therapy.

Two or more modes of therapy as employed herein refers to at least two therapies which have different modes of action and/or different activities and/or different routes of administration.

Unless the context indicated otherwise refractory and resistant are used to interchangeably herein to refer to where the cancer does not respond to therapy or does not responds poorly to therapy.

In some instances patients may benefit from having the initial dose reduced to 300mg or 200mg bi-daily.

Other patients may benefit from receiving the compound of formula (**I**), such as Varlitinib for in a regime which is non-continuous, for example taking medication on alternate days instead of each day or taking medication for four sequential days followed by one, two or three days without medication.

In one embodiment, the salt or free base of compound of formula (**I**) is administered as a pharmaceutical formulation or composition comprising one or more pharmaceutically acceptable excipients.

In one embodiment, the salt or free base (including API) compound of formula (**I**) or pharmaceutical composition thereof is administered orally, for example as tablet or capsule.

In one embodiment the treatment regime for the salt or free base of compound of formula (I) or pharmaceutical composition thereof is commenced before chemotherapy is commenced and is continued until at least the chemotherapy is started, in particular is continued for at least the duration of the chemotherapy.

In one embodiment the treatment regime for the salt or free base (including API) of compound of formula (**I**) is commenced concomitantly with the commencement of the chemotherapy and continued for at least the duration of the chemotherapy.

Thus, in one embodiment the salt or free base (including API) of the compound of formula (**I**) and the chemotherapy are administered on the same day.

Thus, the pharmacology effect of the salt of free base (including API) of the compound of formula (**I**) and the chemotherapy overlap in the patient when the effects of the compound of formula (**I**) can render the cancer cells in more susceptible to chemotherapy than they would be otherwise.

In one embodiment, the chemotherapy is cytotoxic chemotherapy.

In one embodiment, a combination therapy according to the present disclosure comprises a RON inhibitor, for example as disclosed WO2008/058229.

In one embodiment, a combination therapy comprises a checkpoint inhibitor, such as a CTLA4 inhibitor, a PD-1 inhibitor or a PD-L1 inhibitor, in particular an antibody or binding fragment thereof.

Analytical tests for assessing the expression or over expression of HER2 are known and available in the art.

### Cancers

Liver cancer as employed herein refers to cancer of the liver, for example hepatocellular carcinoma including fibrolamellar carcinoma, cholangiocarcinoma, angiosarcoma and hepatoblastoma.

Gastric cancer as employed herein refers to cancer of the stomach, for example squamous cell cancers, lymphoma including non-hodgkin lymphoma, gastrointestinal stromal tumour, or neuroendocrine tumours.

Prostate cancer as employed herein refers to cancer of the prostate, for example ductal adenocarcinoma, transitional cell (urothelial cancer), squamous cell cancer, carcinoid of the prostate, small cell cancer or sarcoma and sarcomatoid cancer.

Pancreatic cancer as employed herein includes exocrine cancers (including rare forms thereof such as cystitic tumours, and cancer of the acinar cells), endocrine pancreatic tumours (including gastrinomas, insulinomas, somatostatinomas, VIPomas, glucagonomas), pancreatoblastoma, sarcomas of the pancreas and lymphoma.

Biliary tract cancer as employed herein refers to cholangiocarcinoma (intrahepatic, extrahepatic), gall bladder cancer and ampullary carcinoma.

In one embodiment, the presently disclosed pharmaceutically acceptable salt is provided for use in the treatment of cholangiocarcinoma.

Colorectal cancer as employed herein refers to cancer or the colon and/or rectum and includes squamous cell cancers, carcinoid tumours, sarcomas and lymphomas.

Breast cancer as employed herein refers to cancer of the breast and includes ductal cardinoma in situ, lobular carcinoma in situ, invasive ductal breast cancer, invasive lobular breast cancer, invasive breast cancer, Paget's disease, angiosarcoma of the breast and rare types of breast cancer such as medullary breast cancer, mucinous breast cancer, tubular breast cancer, adenoid cystic carcinoma of the breast metaplastic breast cancer, basal type breast cancer and papillary breast cancer.

Ovarian cancer as employed herein refers to cancer of an ovary and includes serious, endometrioid, clear cell, mucinous, undifferentiated or unclassified, germline and other rare ovarian tumours such as teratoma of the ovary (mature teratoma and immature teratoma) and borderline ovarian tumours. Epithelia ovarian cancers are serious, endometrioid, clear cell, mucinous and undifferentiated or unclassified.

There are more than 30 different types of ovarian cancer which are classified according to the type of cell from which they start Cancerous ovarian tumours can start from three common cell types:
- Surface Epithelium - cells covering the lining of the ovaries
- Germ Cells - cells that are destined to form eggs, and
- Stromal Cells - Cells that release hormones and connect the different structures of the ovaries.
The present disclosure relates to treatment of ovarian cancer from any source, for example as described herein, in particular epithelium cells. Epithelial ovarian carcinomas (EOCs) account for 85 to 90 percent of all cancers of the ovaries.

**Common Epithelial Tumours** - Epithelial ovarian tumours develop from the cells that cover the outer surface of the ovary. Most epithelial ovarian tumours are benign (noncancerous). There are several types of benign epithelial tumours, including serous adenomas, mucinous adenomas, and Brenner tumours. Cancerous epithelial tumours are carcinomas - meaning they begin in the tissue that lines the ovaries. These are the most common and most dangerous of all types of ovarian cancers. Unfortunately, almost 70 percent of women with the common epithelial ovarian cancer are not diagnosed until the disease is advanced in stage.

There are some ovarian epithelial tumours whose appearance under the microscope does not clearly identify them as cancerous. These are called borderline tumours or tumours of low malignant potential (LMP tumours). The method of the present disclosure includes treatment of the latter.

**Germ Cell Tumours** - Ovarian germ cell tumours develop from the cells that produce the ova or eggs. Most germ cell tumours are benign (non-cancerous), although some are cancerous and may be life threatening. The most common germ cell malignancies are maturing teratomas, dysgerminomas, and endodermal sinus tumours. Germ cell malignancies occur most often in teenagers and women in their twenties. Today, 90 percent of patients with ovarian germ cell malignancies can be cured and their fertility preserved.

**Stromal Tumours** - Ovarian stromal tumours are a rare class of tumours that develop from connective tissue cells that hold the ovary together and those that produce the female hormones, estrogen and progesterone. The most common types are granulosa-theca tumours and Sertoli-Leydig cell tumours. These tumours are quite rare and are usually considered low-grade cancers, with approximately 70 percent presenting as Stage I disease (cancer is limited to one or both ovaries).

**Primary Peritoneal Carcinoma**-The removal of one's ovaries eliminates the risk for ovarian cancer, but not the risk for a less common cancer called Primary Peritoneal Carcinoma. Primary Peritoneal Carcinoma is closely rated to epithelial ovarian cancer (most common type). It develops in cells from the peritoneum (abdominal lining) and looks the same under a microscope. It is similar in symptoms, spread and treatment

### Stages of Ovarian Cancer

Once diagnosed with ovarian cancer, the stage of a tumour can be determined during surgery, when the doctor can tell if the cancer has spread outside the ovaries. There are four stages of ovarian cancer - Stage I (early disease) to Stage IV (advanced disease). The treatment plan and prognosis (the probable course and outcome of your disease) will be determined by the stage of cancer you have.

In one embodiment the ovarian cancer is: type I, for example IA, IB or IC; type II, for example IIA, IIB or IIC; type III, for example IIIA, IIIB or IIIC; or type IV.

The present disclosure relates to treatment of any stage of ovarian cancer, in particular as described herein.

Lung cancers are classified according to histological type and are categorized by the size and appearance of the malignant cells seen by a histopathologist under a microscope. For therapeutic purpose, two broad classes are distinguished: non-small cell lung carcinoma and small cell lung carcinoma.

In one embodiment the epithelial cancer is lung cancer, for example small-cell lung cancer (SCLC) and non-small-cell lung cancer (NSCLC).

**Non-small-cell lung carcinoma**-The three main subtypes of NSCLC are adenocarcinoma, squamous-cell carcinoma and large-cell carcinoma.

Nearly 40% of lung cancers are adenocarcinoma, which usually originates in peripheral lung tissue. A subtype of adenocarcinoma, the bronchioloalveolar carcinoma, is more common in female never-smokers, and may have a better long term survival.

Squamous-cell carcinoma accounts for about 30% of lung cancers. They typically occur close to large airways. A hollow cavity and associated cell death are commonly found at the center of the tumour. About 9% of lung cancers are large-cell carcinoma. These are so named because the cancer cells are large, with excess cytoplasm, large nuclei and conspicuous nucleoli.

**Small-cell lung carcinoma-** In small-cell lung carcinoma (SCLC), the cells contain dense neurosecretory granules (vesicles containing neuroendocrine hormones), which give this tumour an endocrine/paraneoplastic syndrome association. Most cases arise in the larger airways (primary and secondary bronchi). These cancers grow quickly and spread early in the course of the disease. Sixty to seventy percent have metastatic disease at presentation.

In one embodiment the cancer is non-small lung carcinoma.

In one embodiment there is provided treatment of renal cancer, for example renal cell carcinoma and/or urothelial cell carcinoma using an oncolytic adenovirus as disclosed herein. Other examples of renal cancer include squamous cell carcinoma, juxtaglomerular cell tumour (reninoma), angiomyolipoma, renal oncocytoma, Bellini duct carcinoma, clear-cell sarcoma of the kidney, mesoblastic nephroma, Wilms' tumour, mixed epithelial stromal tumour, clear cell adenocarcinoma, transitional cell carcinoma, inverted papilloma, renal lymphoma, teratoma, carcinosarcoma, and carcinoid tumour of the renal pelvis.

In one embodiment the cancer is bladder cancer, for example is any of several types of malignancy arising from the epithelial lining (i.e., the urothelium) of the urinary bladder. About 90% of bladder cancers are transitional cell carcinoma. The other 10% are squamous cell carcinoma, adenocarcinoma, sarcoma, small cell carcinoma, and secondary deposits from cancers elsewhere in the body. The staging of is given below.

**T (Primary tumour)-TX** Primary tumour cannot be assessed; **T0** No evidence of primary tumour; **Ta** Non-invasive papillary carcinoma; **Tis** Carcinoma in situ ('flat tumour'); **T1** Tumour invades subepithelial connective tissue; **T2a** Tumour invades superficial muscle (inner half); **T2b** Tumour invades deep muscle (outer half); **T3** Tumour invades perivesical tissue; **T3a** Microscopically; **T3b** Macroscopically (extravesical mass); **T4a** Tumour invades prostate, uterus or vagina; **T4b** Tumour invades pelvic wall or abdominal wall

**N (Lymph nodes)-NX** Regional lymph nodes cannot be assessed; **N0** No regional lymph node metastasis; **N1** Metastasis in a single lymph node 2 cm or less in greatest dimension; **N2** Metastasis in a single lymph node more than 2 cm but not more than 5 cm in greatest dimension, or multiple lymph nodes, none more than 5 cm in greatest dimension; N3 Metastasis in a lymph node more than 5 cm in greatest dimension

**M (Distant metastasis)-MX** Distant metastasis cannot be assessed; **M0** No distant metastasis; **M1** Distant metastasis.

Thyroid cancer as employed herein refers to cancer of the thyroid originating from follicular or parafollicular thyroid cells and includes papillary thyroid cancer (75% to 85% of cases); follicular thyroid cancer (10% to 20% of cases); medullary thyroid cancer (5% to 8% of cases)- cancer of the parafollicular cells, often part of multiple endocrine neoplasia type 2; poorly differentiated thyroid cancer; anaplastic thyroid cancer (less than 5% of cases) is not responsive to treatment and can cause pressure symptoms, thyroid lymphoma, squamous cell thyroid carcinoma, sarcoma of thyroid.

Renal cancer as employed herein refers to cancer of the kidney, for example renal cell carcinoma and transitional cell carcinoma of the renal pelvis, such as squamous cell carcinoma, juxtaglomerular cell tumor (reninoma), angiomyolipoma, renal oncocytoma, bellini duct carcinoma, clear-cell sarcoma of the kidney, mesoblastic nephroma, Wilms' tumor, mixed epithelial stromal tumor, clear cell adenocarcinoma, transitional cell carcinoma, inverted papilloma, renal lymphoma, teratoma, carcinosarcoma; carcinoid tumor of the renal pelvis.

Bladder cancer as employed herein refers to cancer of the bladder including transitional cell bladder cancer, carcinoma in situ, papillary cancer and rarer types of bladder cancer such as squamous cell cancer and adenocarcinoma.

Esophageal cancer as employed herein refers to cancer of the oesphagus including esophageal squamous-cell carcinomas, esophageal adenocarcinomas, and variants of squamous-cell carcinoma, and non-epithelial tumors, such as leiomyosarcoma, malignant melanoma, rhabdomyosarcoma, lymphoma, among others.

Head and neck cancer as employed herein refers to cancer of the neck and/or head, including mouth cancer, nasopharyngeal cancer, oropharyngeal cancer, paranasal sinus cancer and salivary gland cancer.

### Chemotherapeutic Agents

Chemotherapeutic agent and chemotherapy or cytotoxic agent are employed interchangeably herein unless the context indicates otherwise.

Chemotherapy as employed herein is intended to refer to specific antineoplastic chemical agents or drugs that are "selectively" destructive to malignant cells and tissues, for example alkylating agents, antimetabolites including thymidylate synthase inhibitors, anthracyclines, anti-microtubule agents including plant alkaloids, topoisomerase inhibitors, parp inhibitors and other antitumour agents. Selectively in this context is used loosely because of course many of these agents have serious side effects.

The preferred dose may be chosen by the practitioner, based on the nature of the cancer being treated.

In one embodiment a therapy of the present disclosure comprises an alkylating agents, for example nitrogen mustards, nitrosoureas, tetrazines, aziridines, platins and derivatives, and non-classical alkylating agents.

In one embodiment a therapy of the present disclosure comprises a platinum containing chemotherapeutic agent (also referred to as platins), such as cisplatin, carboplatin, oxaliplatin, satraplatin, picoplatin, nedaplatin, triplatin and lipoplatin (a liposomal version of cisplatin), in particular cisplatin, carboplatin and oxaliplatin.

The dose for cisplatin ranges from about 20 to about 270 mg/m² depending on the exact cancer. Often the dose is in the range about 70 to about 100mg/m².

In on embodiment a therapy of the present disclosure comprises a nitrogen mustard, for example mechlorethamine, cyclophosphamide, melphalan, chlorambucil, ifosfamide and busulfan.

In on embodiment a therapy of the present disclosure comprises a nitrosourea, for example N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide.

In on embodiment a therapy of the present disclosure comprises an aziridines, for example thiotepa, mytomycin and diaziquone (AZQ).

In on embodiment a therapy of the present disclosure comprises an antimetabolites, for example anti-folates (for example methotrexate and pemetrexed), purine analogues (for example thiopurines, such as azathiopurine, mercaptopurine, thiopurine, fludarabine (including the phosphate form), pentostatin and cladribine), pyrimidine analogues (for example fluoropyrimidines, such as 5-fluorouracil and prodrugs thereof such as capecitabine [Xeloda^{®}]), floxuridine, gemcitabine, cytarabine, decitabine, raltitrexed(tomudex) hydrochloride, cladribine and 6-azauracil.

In on embodiment a therapy of the present disclosure comprises a pyrimidine analogue, for example 5-fluorouracil and prodrugs thereof such as capecitabine [Xeloda^{®}]), floxuridine, gemcitabine, cytarabine, decitabine, raltitrexed(tomudex) hydrochloride, cladribine and 6-azauracil.

In on embodiment a therapy of the present disclosure comprises an anthracyclines, for example daunorubicin (Daunomycin), daunorubicin (liposomal), doxorubicin (Adriamycin), doxorubicin (liposomal), epirubicin, idarubicin, valrubicin currenlty used only to treat bladder cancer and mitoxantrone an anthracycline analog, in particular doxorubicin.

In on embodiment a therapy of the present disclosure comprises an anti-microtubule agents, for example vinca alkaloids and taxanes.

In on embodiment a therapy of the present disclosure comprises a vinca alkaloids, for example completely natural chemicals for example vincristine and vinblastine and also semisynthetic vinca alkaloids, for example vinorelbine, vindesine, and vinflunine.

In on embodiment a therapy of the present disclosure comprises a Taxane, for example paclitaxel, docetaxel, abraxane, carbazitaxel and derivatives of thereof. Derivatives of taxanes as employed herein includes reformulations of taxanes like taxol, for example in a micelluar formulaitons, derivatives also include chemical derivatives wherein synthetic chemistry is employed to modify a starting material which is a taxane.

In on embodiment a therapy of the present disclosure comprises a topoisomerase inhibitors, for example type I topoisomerase inhibitors, type II topoisomerase inhibitors and type II topoisomerase poisons. Type I inhibitors include topotecan, irinotecan, indotecan and indimitecan. Type II inhibitors include genistein and ICRF 193 which has the following structure:

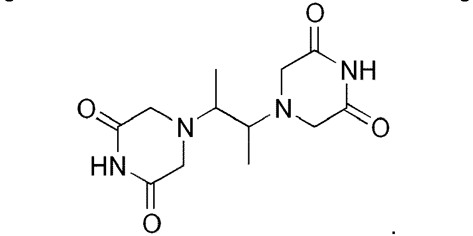

Type II poisons include amsacrine, etoposide, etoposide phosphate, teniposide and doxorubicin and fluoroquinolones.

In one embodiment the chemotherapeutic is a PARP inhibitor.

### Combination Therapy

In one embodiment the chemotherapy comprises a combination of chemotherapy agents, in particular cytotoxic chemotherapeutic agents, for example a pyrimidine analogue and a platin.

In one embodiment a combination of chemotherapeutic agents employed is, for example a platin and 5-FU or a prodrug thereof, for example cisplatin or oxaplatin and capecitabine or gemcitabine, such as FOLFOX.

In one embodiment the chemotherapy combination comprises a platin, such as cisplatin and fluorouracil or capecitabine.

In one embodiment the chemotherapy combination in capecitabine and oxaliplatin (Xelox).

In one embodiment the chemotherapy is a combination of folinic acid and 5-FU, optionally in combination with oxaliplatin.

In one embodiment the chemotherapy is a combination of folinic acid, 5-FU and irinotecan (FOLFIRI), optionally in combination with oxaliplatin (FOLFIRINOX). The regimen consists of: irinotecan (180 mg/m² IV over 90 minutes) concurrently with folinic acid (400 mg/m² [or 2 × 250 mg/m²] IV over 120 minutes); followed by fluorouracil (400-500 mg/m² IV bolus) then fluorouracil (2400-3000 mg/m² intravenous infusion over 46 hours). This cycle is typically repeated every two weeks. The dosages shown above may vary from cycle to cycle.

In one embodiment the chemotherapy combination employs a microtubule inhibitor, for example vincristine sulphate, epothilone A, N-[2-[(4-Hydroxyphenyl)amino]-3-pyridinyl]-4-methoxybenzenesulfonamide (ABT-751), a taxol derived chemotherapeutic agent, for example paclitaxel, abraxane, or docetaxel or a combination thereof.

In one embodiment the therapy according to the present disclosure, such as a chemotherapy combination comprises an mTor inhibitor. Examples of mTor inhibitors include: everolimus (RAD001), WYE-354, KU-0063794, papamycin (Sirolimus), Temsirolimus, Deforolimus(MK-8669), AZD8055 and BEZ235(NVP-BEZ235).

In one embodiment the therapy according to the present disclosure, such as a chemotherapy combination comprises a MEK inhibitor. Examples of MEK inhibitors include: AS703026, CI-1040 (PD184352), AZD6244 (Selumetinib), PD318088, PD0325901, AZD8330, PD98059, U0126-EtOH, BIX 02189 or BIX 02188.

In one embodiment the therapy according to the present disclosure, such as a chemotherapy combination comprises an AKT inhibitor. Examples of AKT inhibitors include: MK-2206 and AT7867.

In one embodiment a combination therapy comprises an aurora kinase inhibitor. Examples of aurora kinase inhibitors include: Aurora A Inhibitor I, VX-680, AZD1152-HQPA (Barasertib), SNS-314 Mesylate, PHA-680632, ZM-447439, CCT129202 and Hesperadin.

In one embodiment the therapy according to the present disclosure, such as a chemotherapy combination employs a p38 inhibitor, for example as disclosed in WO2010/038086, such as N-[4-({4-[3-(3-*tert*-Butyl-1-*p*-tolyl-1*H*-pyrazol-5-yl)ureido]naphthalen-1-yloxy}methyl)pyridin-2-yl]-2-methoxyacetamide.

In one embodiment the combination employs a Bcl-2 inhibitor. Examples of Bcl-2 inhibitors include: obatoclax mesylate, ABT-737, ABT-263(navitoclax) and TW-37.

In one embodiment the therapy according to the present disclosure, such as a chemotherapy combination comprises an antimetabolite such as capecitabine (xeloda), fludarabine phosphate, fludarabine (fludara), decitabine, raltitrexed (tomudex), gemcitabine hydrochloride and cladribine.

In one embodiment the therapy according to the present disclosure, such as a chemotherapy combination comprises ganciclovir, which may assist in controlling immune responses and/or tumour vasculation.

In one embodiment the therapy according to the present disclosure, such as a chemotherapy includes a PARP inhibitor.

In one embodiment the therapy according to the present disclosure, such as a chemotherapy includes an inhibitor of cancer metabolism with specific inhibition of the activity of the DHODH enzyme.

In one embodiment one or more therapies employed in the method herein are metronomic, that is a continuous or frequent treatment with low doses of anticancer drugs, often given concomitant with other methods of therapy.

In one embodiment, there is provided the use of multiple cycles of treatment (such as chemotherapy) for example 2, 3, 4, 5, 6, 7, 8.

In one embodiment the chemotherapy is employed in a 28 day cycle.

### Pharmaceutical compositions/Formulations

In another aspect, the present disclosure provides a pharmaceutical composition comprising a pharmaceutically acceptable salt of the present disclosure and pharmaceutically acceptable carriers and/or diluents thereof, and if desired, other active ingredients, which may be administered orally, intravascularly, subcutaneously, intramuscularly or topically for the use as anticoagulant in a mammal in need thereof.

The present invention also provides a process for preparation of a pharmaceutical or diagnostic composition comprising adding and mixing the salt or free base of the present invention together with one or more of a pharmaceutically acceptable excipient, diluent or carrier.

The salt or free base of the disclosure may be the sole active ingredient in the pharmaceutical or diagnostic composition or may be accompanied by other active ingredients, such as further chemotherapeutic agents as described above.

The pharmaceutical compositions suitably comprise a therapeutically effective amount of the molecule of the invention. The term "therapeutically effective amount" as used herein refers to an amount of a therapeutic agent needed to treat, ameliorate or prevent a targeted disease or condition, or to exhibit a detectable therapeutic or preventative effect The therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgment of the clinician. Pharmaceutical compositions may be conveniently presented in unit dose forms containing a predetermined amount of an active agent of the invention per dose.

Compositions may be administered individually to a patient or may be administered in combination (e.g. simultaneously, sequentially or separately) with other agents, drugs or hormones.

The dose at which the salt or free base of the present invention is administered depends on the nature of the condition to be treated, for example the extent of the disease/inflammation present and on whether the molecule is being used prophylactically or to treat an existing condition.

Pharmaceutically acceptable carriers in therapeutic compositions may enable the pharmaceutical compositions to be formulated as tablets, pills, dragees, capsules, liquids, gels, syrups, slurries and suspensions, for ingestion by the patient.

Suitable forms for administration include forms suitable for parenteral administration, e.g. by injection or infusion, for example by bolus injection or continuous infusion.

Once formulated, the compositions of the invention can be administered directly to the subject. The subjects to be treated can be animals. However, in one or more embodiments the compositions are adapted for administration to human subjects.

The pharmaceutical compositions of this invention may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes.

Hyposprays may also be used to administer the pharmaceutical compositions of the invention. Typically, the therapeutic compositions may be prepared as injectables, either as liquid solutions or suspensions. Solid forms (for example lyophilised forms) suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion. Dosage treatment may be a single dose schedule or a multiple dose schedule.

It will be appreciated that the active ingredient in the composition will be a chemical molecule. As such, it may be susceptible to degradation in the gastrointestinal tract. Thus, if the composition is to be administered by a route using the gastrointestinal tract, the composition will need to contain agents which protect the binding protein from degradation but which release the molecule once it has been absorbed from the gastrointestinal tract.

A thorough discussion of pharmaceutically acceptable carriers is available in Remington's Pharmaceutical Sciences (Mack Publishing Company, N.J. 1991).

In one embodiment the formulation is provided as a formulation for topical administrations including inhalation.

Suitable inhalable preparations include inhalable powders, metering aerosols containing propellant gases or inhalable solutions free from propellant gases.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

The propellant gases which can be used to prepare the inhalable aerosols are known in the art Suitable propellant gases are selected from among hydrocarbons such as n-propane, n-butane or isobutane and halohydrocarbons such as chlorinated and/or fluorinated derivatives of methane, ethane, propane, butane, cyclopropane or cyclobutane. The above-mentioned propellant gases may be used on their own or in mixtures thereof. Particularly suitable propellant gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellant gas-containing inhalable aerosols may also contain other ingredients, such as cosolvents, stabilisers, surface-active agents (surfactants), antioxidants, lubricants and means for adjusting the pH. All these ingredients are known in the art.

The propellant gas-containing inhalable aerosols according to the invention may contain up to 5 % by weight of active substance. Aerosols according to the invention contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active ingredient

Alternatively topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

Nebulisable formulation according to the present disclosure may be provided, for example, as single dose units (e.g., sealed plastic containers or vials) packed in foil envelopes. Each vial contains a unit dose in a volume, e.g., 2 mL, of solvent/solution buffer.

"Comprising" in the context of the present specification is intended to mean "including". Where technically appropriate, embodiments of the invention may be combined.

Embodiments are described herein as comprising certain features/elements. The disclosure also extends to separate embodiments consisting or consisting essentially of said features/elements.

Any embodiments specifically and explicitly recited herein may form the basis of a disclaimer either alone or in combination with one or more further embodiments.

Embodiments explicitly disclosed herein may be combined as technically appropriate.

The invention will now be described with reference to the following examples, which are merely illustrative and should not in any way be construed as limiting the scope of the present invention.

### BRIEF SUMMARY OF THE FIGURES

- **Figure 1**: Graph showing the results of X-ray Powder Diffraction (XRPD) analysis of Varlitinib

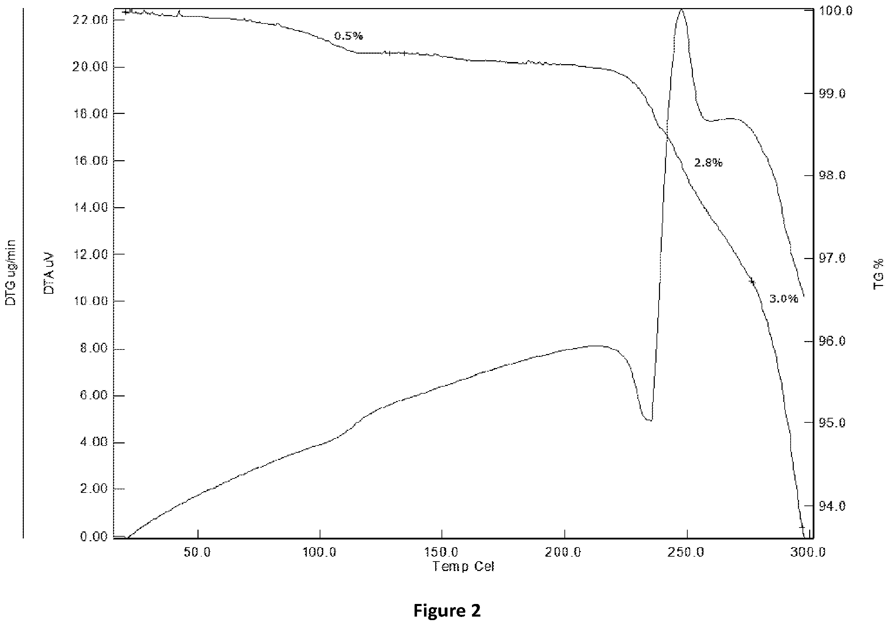

- **Figure 2**: Graph showing results of Thermogravimetric Analysis (TG/DTA) analysis of varlitinib
- **Figure 3**: Graph showing the results of NMR analysis of Varlitinib
- **Figure 4**: Shows XRPD analsyis results from the primary salt screen

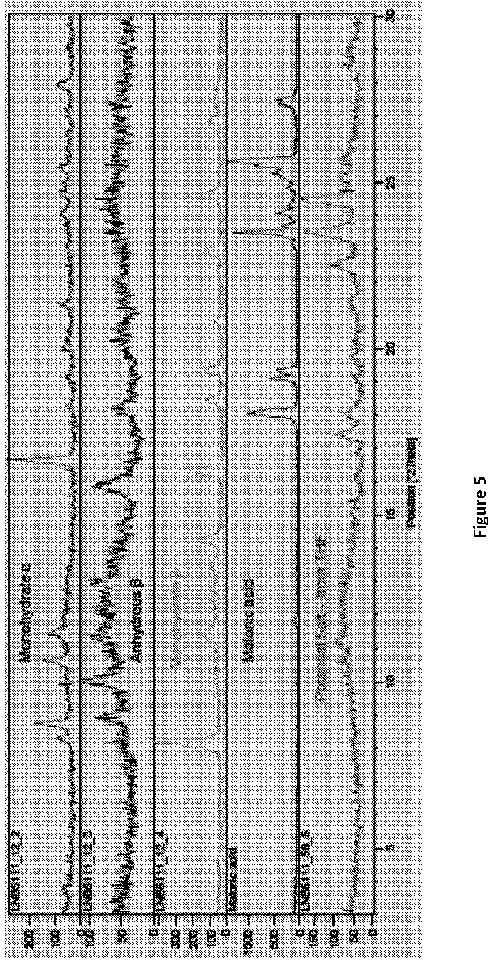

- **Figure 5**: Graph showing the results of the primary salt screen data for malonic acid

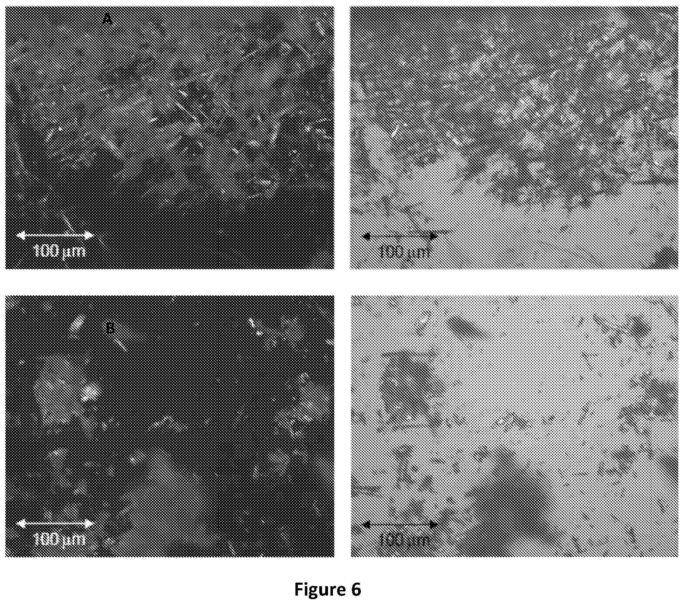

- **Figure 6**: PLM analysis images of a malonic acid salt of Varlitinib taken using polarised lenses and non-polarised lenses. (A) from acetone and (B) from THF
- **Figure 7**: Graph showing the results of XRPD analysis of malonate salt of Varlitinib
- **Figure 8**: PLM analysis images for malonate salt of Varlitinib taken using polarised lenses and non-polarised lenses
- **Figure 9**: Graph showing results of TG/DTA analysis for malonate salt of Varlitinib
- **Figure 10**: Graph showing results of DSC analysis for malonate salt of Varlitinib
- **Figure 11**: Graph showing results of DVS analysis for malonate salt of Varlitinib
- **Figure 12**: Graph showing results of post DVS XRPD analysis of malonate salt of Varlitinib
- **Figure 13**: Graph showing results of NMR analysis of malonate salt of Varlitinib
- **Figure 14**: Graph showing results of purity analysis by HPLC of malonate salt of Varlitinib
- **Figure 15**: Graph showing results of storage testing of malonate salt of Varlitinib
- **Figure 16**: Graph showing results of hydration study of malonate salt of Varlitinib
- **Figure 17**: Graph showing results of disproportionation study of malonate salt of Varlitinib
- **Figure 18**: Graph showing results of thermodynamic solubility analysis of malonate salt of Varlitinib
- **Figure 19**: Graph showing results of aqueous solubility analysis of malonate salt of Varlitinib
- **Figure 20**: Graph showing results of purity analysis of malonate salt of Varlitinib. Black line shows purity of Varlitinib. Grey line shows % water content

### EXAMPLES

### Example 1 - Experimental techniques used

### X-ray Powder Diffraction (XRPD)

XRPD analysis was carried out on a Siemens D5000, scanning the samples between 3 and 30 °2-theta. The samples were gently compressed on a glass disc inserted into the sample holder. The sample was then loaded into a Siemens D5000 diffractometer running in reflection mode and analysed, using the following experimental conditions:

| Raw Data Origin | Siemens-binary V2 (RAW) |
|---|---|
| Start Position [°2Th.] | 3.0000 |
| End Position [°2Th.] | 3.0000 |
| Step Size [°2Th.] | 0.0200 |
| Scan Step Time [s] | 1 |
| Scan Type | Continuous |
| Offset [°2Th.] | 0.0000 |
| Divergence Slit Type | Fixed |
| Divergence Slit Size [°] | 2.0000 |
| Specimen Length [mm] | various |
| Receiving Slit Size [mm] | 0.2000 |
| Measurement Temperature [°C] | 20.00 |
| Anode Material | Cu |
| K-Alphal [Å] | 1.54060 |
| K-Alpha2 [Å] | 1.54443 |
| K-Beta [Å] | 1.39225 |
| K-A2 / K-A1 Ratio | 0.50000 (nominal) |
| Generator Settings | 40 Ma, 40kV |
| Diffractometer Type | d5000 |
| Diffractometer Number | 0 |
| Goniometer Radius [mm] | 217.50 |
| Incident Beam Monochromator | No |
| Diffracted Beam Monochromator | (Graphite) |
| Spinning | No |

### Polarised light Microscopy (PLM)

The presence of crystallinity (birefringence) was determined using an Olympus BX50 polarising microscope, equipped with a Motic camera and image capture software (Motic Images Plus 2.0). All images were recorded using the 20x objective, unless otherwise stated.

### Thermogravimetric Analysis (TGA)

Approximately, 5 mg of material was accurately weighed into an open aluminium pan and loaded into a simultaneous thermogravimetric/differential thermal analyser (TG/DTA) and held at room temperature. The sample was then heated at a rate of 10°C/min from 25°C to 30 time the change in sample weight was recorded along with any differential thermal events (DTA). Nitrogen was used as the purge gas, at a flow rate of 100 cm³/min.

### Differential Scanning Calorimetry (DSC)

Approximately, 5 mg of material was weighed into an aluminium DSC pan and sealed non-hermetically with a pierced aluminium lid. The sample pan was then loaded into a Seiko DSC6200 (equipped with a cooler). The sample and reference were heated to *ca*. 220°C at a scan rate of 10°C/min and the resulting heat flow response monitored.

### ¹H Nuclear Magnetic Resonance (¹H-NMR)

¹H-NMR experiments were performed on a Bruker AV400 (frequency: 400 MHz) ¹H experiments of each sample were performed in deuterated DMSO and each sample was prepared to *ca*. 10 mM concentration.

### Infrared Spectroscopy (IR)

Infrared spectroscopy was carried out on a Bruker ALPHA P spectrometer. Sufficient material was placed onto the centre of the plate of the spectrometer and the spectra were obtained using the following parameters:

| | |
|---|---|
| Resolution | 4cm⁻¹ |
| Background Scan Time | 16 scans |
| Sample Scan Time | 16 scans |
| Data Collection | 4000 to 400 cm⁻¹ |
| Result Spectrum | Transmittance |
| Software | OPUS version 6 |

### Dynamic Vapour Sorption (DVS)

Approximately 10 mg of sample was placed into a mesh vapour sorption balance pan and loaded into a DVS-1 dynamic vapour sorption balance by Surface Measurement Systems. The sample was subjected to a ramping profile from 20 - 90% relative humidity (RH) at 10% increments maintaining the sample at each step until a stable weight had been achieved (99.5 % step completion). After completion of the sorption cycle, the sample was dried using the same procedure, but all the way down to 0 % RH and finally taken back to the starting point of 20% RH. The weight change during the sorption/desorption cycles were plotted, allowing for the hygroscopic nature of the sample to be determined.

### Karl Fischer Coulometric Titration (KF)

*ca*. 10-15 mg of solid material was accurately weighted into a vial. The solid was then manually introduced into the titration cell of a Mettler Toledo C30 Compact Titrator. The vial was back-weighed after the addition of the solid and the weight of the added solid entered on the instrument Titration was initiated once the sample had fully dissolved in the cell. The water content was calculated automatically by the instrument as a percentage and the data printed.

### High Performance Liquid Chromatography-Ultraviolet Detection (HPLC-UV)

The method and column detailed below were supplied by Array Bioharma. Inc.

| Instrument | Agilent 1100 HPLC |
|---|---|
| Column | YMC ODS-AQ (4.6 × 150nm, 3mm) |
| Column Temperature | 30°C |
| UV wavelength | 245nm |
| Injection Volume | 10µl |
| Flow Rate | 1.0ml/min |
| Mobile Phase A | 0.01% TFA in H₂0 |
| Mobile Phase B | Acetonitrile |

The following gradient program shown in Table 1 was used:

**Table 1 - Gradient program used for HPLC-UV**

| | | | | | | | | |
|---|---|---|---|---|---|---|---|---|
| **Time (Mins)** | **0** | **1** | **15** | **20** | **21** | **22** | **23** | **30** |
| **Solvent B [%]** | **2** | **2** | **35** | **65** | **95** | **95** | **2** | **2** |

### EXAMPLE 2 - Methodology for Primary Salt Screen

### Preparation of Varlitinib for salt screen

Varlitinib was placed into a vacuum oven set at *ca*. 80 °C and dried for a minimum of 3 hours at *ca*. 75 mbar. This method was confirmed to prepare the anhydrous α form upon analysis of the dried material, the results of which are shown below.

All solvents used throughout the project were dried using molecular sieves (3A, 0.4-0.8 mm beads).

### Selection of counterions and solvents

The counterions and solvent used for the screens are shown in Tables 2 and 3 respectively:

**Table 2 - Counterions**

| **Counterions** |
|---|
| Adipic acid |
| Benzene sulphonic acid |
| Benzoic acid |
| Citric acid |
| 2,5-Dihydroxybenzoic acid |
| Ethane disulphonic acid |
| Fumaric acid |
| Galataric acid (Mucic acid) |
| 1-Hydroxy-2-napthoic acid |
| Maleic acid |
| Malic acid |
| Malonic acid |
| 1,5-Napthelene disulphonic acid |
| Oxalic acid |
| Succinic acid |
| DL-Tartaric acid |

**Table 3- Solvents**

| **Solvents** |
|---|
| Acetone |
| Diisopropyl Ether |
| Ethyl Acetate |
| Methanol (MEK*) |
| Tetrahydrofuran |
| Acetonitrile:Water (5%) |

| |
|---|
| *MEK used in place of methanol for sulphonic acids |

### Preparation of salt screening experiments

For each counterion and solvent (16 counterions and 1 blank row, in each of 6 solvents), *ca.* 25 mg of dried varlitinib was slurried in 300 µl of allocated solvent and mixed with the equivalent mass of acid, also dissolved in the allocated solvent. If the acid was insoluble in a selected solvent, a slurry was used instead.

### Temperature cycling

Mixtures of dried varlitinib/counterion/solvent prepared were temperature cycled to 40 °C in 4 hour cycles of a minimum of 24 hours (the cooling/heating rates after the 4 hour periods were up was *ca*. 1 °C/min). Any solids present were isolated and allowed to dry at ambient conditions prior to analysis.

### EXAMPLE 3 - Methodology for Secondary Salt Screen

### Scale-up of salt forms

For each counterion selected, *ca*. 300 mg of ASLAN001 (dried as described in Example 2) was slurried in 12 volumes of the selected solvent and mixed with the equivalent mass of acid, also dissolved in the chosen solvent. If the acid was insoluble in the selected solvent, a slurry was used. The mixtures of dried ASLAN001/counterion/solvent were then temperature cycled between RT and 40°C (4 hour cycles) for ca. 4 days.

Table 4 shows the counterion and solvent systems selected for the secondary screen:

**Table 4 - Secondary salt screen selections**

| **Counterion** | **Equiv.** | **Solvent** |
|---|---|---|
| Adipic acid | 1 | Ethyl acetate |
| Galataric acid | 1 | Methanol |
| Maleic acid | 1 | Acetonitrile:Water (5%) |
| Malonic acid | 1 | THF |

### Stability Testing

Each potential salt was exposed to environments of 40°C/75%RH, elevated temperature (80°C) and ambient light (*ca*. 22°C in a closed vial), for 1 week to determine stability. Resulting solids were analysed by XRPD to establish if any changes had occurred.

### Salt Disproportionation Studies

Each potential salt was slurried in water at room temperature (*ca*. 22°C), with the excess solid taken at 1, 24 & 48 hours and analysed by XRPD. The pH of the supernatant was also monitored.

### Hydration Studies

Slurries of each potential salt were created in acetone:water mixtures (3, 5 and 10% water) and shaken for *ca*. 48 hours. The resulting solid was then analysed by XRPD to determine if any changes had occurred on slurrying.

### Thermodynamic Solubility

Slurries of each potential salt were created in media of pH 1; 4.5 and 6.8 and shaken for *ca*. 48hours. The resulting solution was then analysed by HPLC and the concentration of material dissolved calculated. Remaining solids were analysed by XRPD to determine if any changes had occurred on slurrying.

### Aqueous Solubility

Slurries of each potential salt were created in deionised water and agitated for 4 and 24 hours at room temperature. After both 4 and 24 hours, the resulting solutions were analysed by HPLC and the concentration of material dissolved calculated. Remaining solids were analysed by XRPD to determine if any changes had occurred on slurrying.

### EXAMPLE 4 - Results of analysis of dried Varlitinib

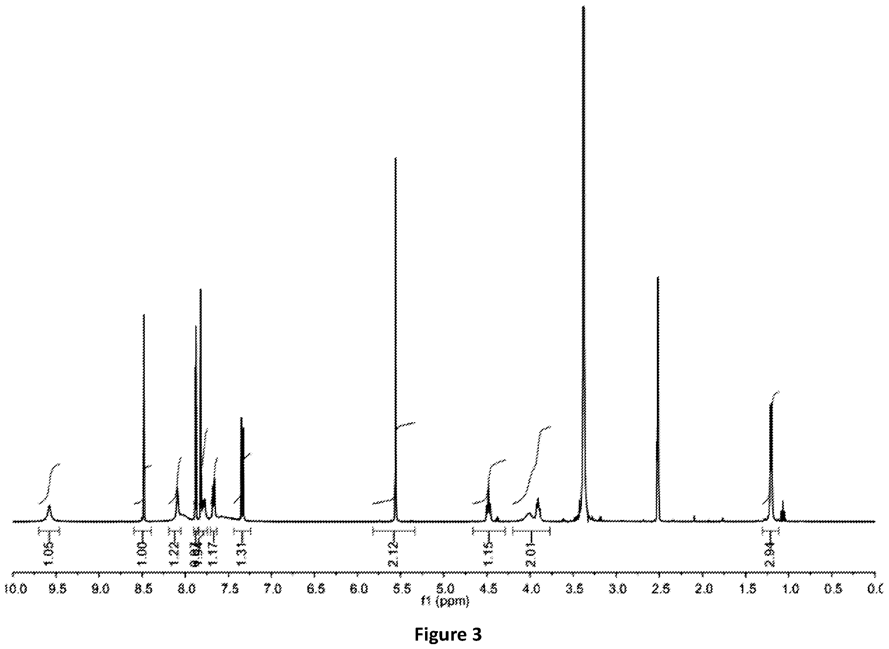

- The XRPD analysis (Figure 1) shows that varlitinib is of a different crystalline form to the monohydrate, consistent with that of the anhydrous α form.
- KF analysis showed that the water content of the material is 0.85%.
- TGA showed an initial loss of 0.5% below 120 °C, with the onset of degradation occurring at *ca*. 230 °C.
- NMR trace is shown in Figure 3.

### EXAMPLE 5 - Results of Primary Salt Screen

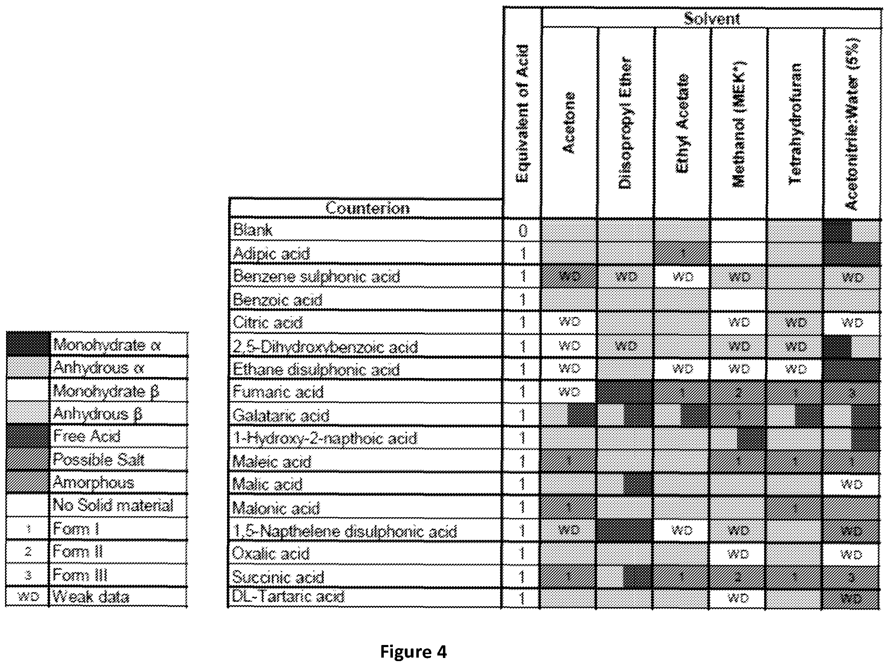

The results based on XRPD for solids obtained in the primary salt screen carried out on dried varlitinib are shown in Figure 4B, which shows XRPD analysis results for the primary salt screen. Based on the results of the primary salt screen, 9 potential salts were identified having the following counterions:
- Adipic acid
- Fumaric acid (possibly 3 forms)
- Galactaric acid
- Maleic acid
- Malonic acid
- Succinic acid (possibly 3 forms)
- Benzene sulfonic acid, naphthalenedisulfonic acid and tartaric acid also showed potential salts but the data was weak
4 potential salts were selected to be scaled up for a secondary screen. The counterions and solvents selected are shown below in Table 5 below:

**Table 5 - Counterion and solvents for secondary salt screen**

| **Counterion** | **Equiv.** | **Solvent** |
|---|---|---|
| Adipic acid | 1 | Ethyl acetate |
| Galataric acid | 1 | Methanol |
| Maleic acid | 1 | Acetonitrile:Water (5%) |
| Malonic acid | 1 | THF |

### EXAMPLE 6 - Results of Secondary Salt Screen

### Example 6A Adipate salt

The adipate salt was prepared in ethyl acetate, using the procedure detailed in Example 3.
- XRPD analysis shows the material to be crystalline and of the same form as produced during the primary screen.
- NMR analysis confirmed salt formation and an APLacid ratio of 1:0.9
- Purity analysis by HPLC showed a purity of 97.12%, which was a slightly lower purity than that of the starting material (97.82%).
- TG/DTA shows a 0.5% weight loss <150 °C, with onset of degradation *ca*. 160 °C,
- DSC analysis showed no thermal events <150°C. An event was observed at 153.9°C, potentially due to the onset of degradation.
- KF analysis showed that the material contained 0.7% water.
- DVS analysis showed the material to uptake 0.27% water between 20-70% RH and 0.42% water overall. No change to the solid form was observed when analysed by XRPD, post DVS.
- Storage testing showed the material to remain unchanged during all 3 types of storage: ambient light, 40°C/75%RH and 80°C.
- Salt disproportionation studies showed there to be a possible form change of adipate salt on slurrying in water after *ca*. 48 hours.
- Hydration studies (i.e. slurry in Acetone:Water (3, 5 and 10% water)) showed the material to have changed after slurrying, indicating a potential hydration risk.
- Thermodynamic solubility results showed:
   - pH1 1.2613 mg/ml
   - pH 4.5 0.0022 mg/ml
   - pH 6.8 <4.45x10⁻⁴ mg/ml

A change in solid form was observed by XRPD from pH1 and pH4.5, indicating potential salt formation.
- Aqueous solubility results showed the following:
   - 4 hours 0.1525 mg/ml
   - 24 hours 0.3573 mg/ml

Solid material was observed to remain unchanged after slurrying.

### Example 6B Mucate salt (unstable)

The mucate salt was prepared in ethyl acetate, using the procedure detailed in Example 3.
- XRPD analysis shows the material to be crystalline and of the same form as produced during the primary screen.
- NMR analysis confirmed salt formation and showed that the API: Acid ratio was 1:1.26
- Purity analysis by HPLC showed a purity of 98.67%, which was higher than that of the starting material (97.82%).
- TG/DTA showed a 5.7% weight loss <150°C, possibly due to unbound solvent or water. The results are inconsistent with KF analysis. Onset of degration at *ca* 160 °C.
- DSC analysis shows a broad endotherm <150°C consistent with loss observed by TGA.

An event was observed at 157.5°C, potentially due to onset of degradation.
- KF analysis showed the material to contain 3.8 % water.
- DVS analysis showed the material to have an initial water content of 3.7%, uptaking 1.58% water between 20-70%RH and 2.25% overall. Subsequent XRPD analysis showed a change in form following exposure to higher levels of humidity.
- Storage testing showed the material to remain unchanged during each of the storage conditions.
- Salt disproportionation studies showed the presence of free acid in the XRPD diffractograms after slurrying in water for 1 and 24 hours. However, after 48 hours the presence of counterion was reduced.
- Hydration studies (i.e. slurry in Acetone:Water mixtures (3, 5 and 10% water)) showed the salt to have disproportionated, with the counterion clearly present in each XRPD diffractogram.
- Thermodynamic solubility results showed:
   - pH1 0.7856 mg/ml
   - pH 4.5 <4.45×10⁻⁴mg/ml
   - pH 6.8 <4.45×10⁻⁴mg/ml

A change in solid form was observed by XRPD from pH1 and pH4.5, indicating potential salt formation.
- Aqueous solubility results showed the following:
   - 4 hours 0.0556 mg/ml
   - 24 hours 0.0337 mg/ml

Solid material was seen to have disproportionated, with counterion clearly present after both 4 and 24 hours.

Disproportionation is a specific type of redox reaction in which an element from a reaction undergoes both oxidation and reduction to form two different products.

### Example 6C Maleate salt (lower purity)

The maleate salt was prepared in acetone, using the procedure detailed in Example 3
- XRPD analysis shows the material to be crystalline and of the same form as produced during the primary screen.
- NMR analysis confirmed salt formation and the ratio of API: Acid was 1:1.
- Purity analysis by HPLC showed a purity of 93.75%, which was lower than that of the starting material (97.82%).
- TG/DTA shows a 3.7% weight loss <150°C, possibly due to unbound solvent or water. The results are inconsistent with KF analysis. Onset of degradation was observed at ca. 150°C.
- DSC analysis showed a broad endotherm <150°C consistent with loss observed by TGA. An event observed at the onset of degradation.
- KF analysis showed the material contained 1.8 % water.
- DVS analysis showed the material to have an initial water content of 2.6%, uptaking 1.95% water between 20-70%RH and 2.84% water overall. When analysed by XRPD, the material was observed to remain unchanged during exposure to varying %RH.
- Storage testing showed the material to remain unchanged during all 3 types of storage: ambient light, 40°C/75%RH and 80°C.
- Salt disproportionation studies showed there to be no change in form of the maleate salt on slurrying in water at 1 hr and 24 hrs. After 48 hrs the material was observed to be predominantly amorphous.
- Hydration studies (i.e. slurry in Acetone:Water mixtures (3, 5 and 10% water)) indicated that the material remained unchanged after slurrying, indicating a low hydration risk.
- Thermodynamic solubility results showed:
   - pH1 1.2138 mg/ml
   - pH 4.5 0.0036 mg/ml
   - pH 6.8 <4.45×10⁻⁴ mg/ml

A change in solid form was observed by XRPD from pH1 and pH4.5, indicating potential salt formation.
- Aqueous solubility results showed the following:
   - 4 hours 0.2570 mg/ml
   - 24 hours 0.4089 mg/ml

Solid material was seen to have remained unchanged after slurrying, with a potential increase in amorphous content

### Example 6D Malonate salt (good stability and good purity)

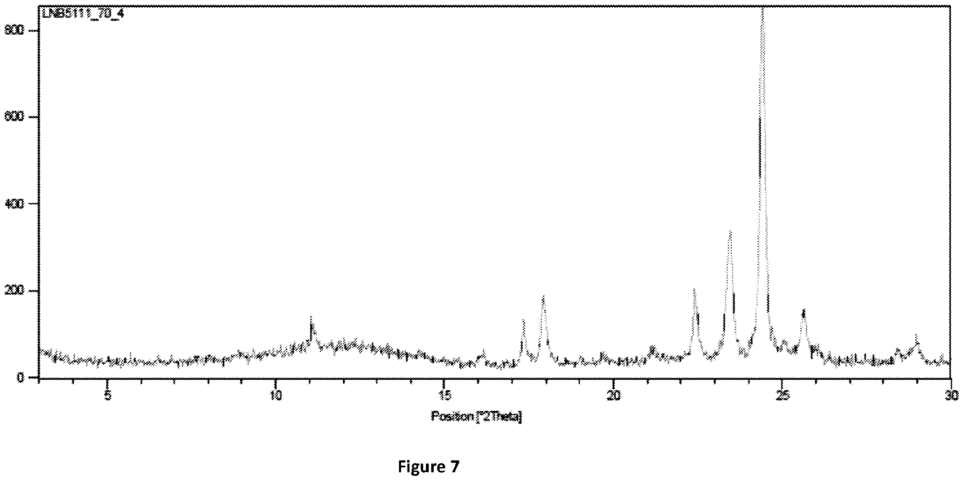

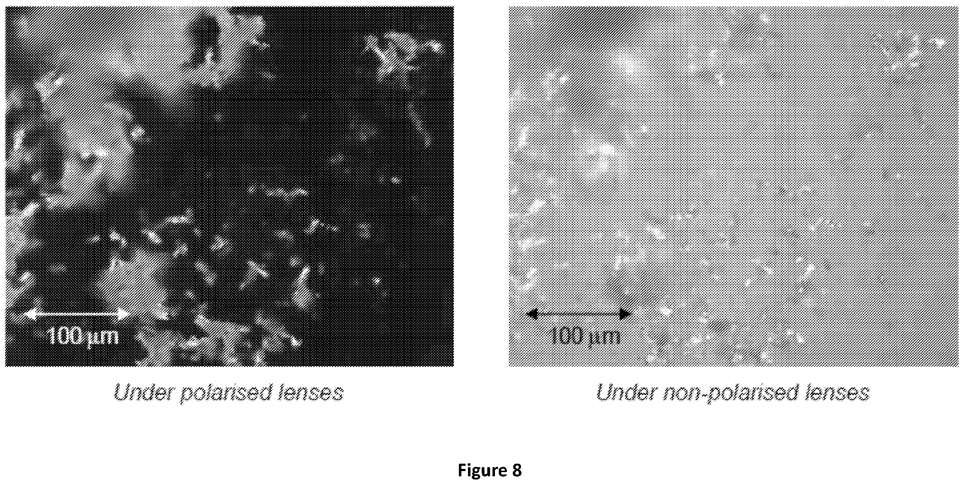

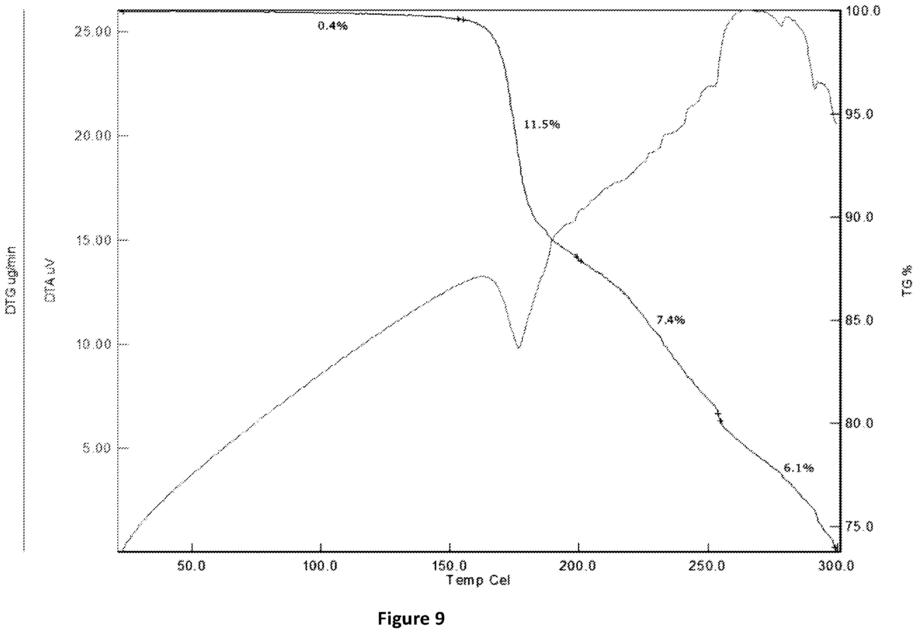

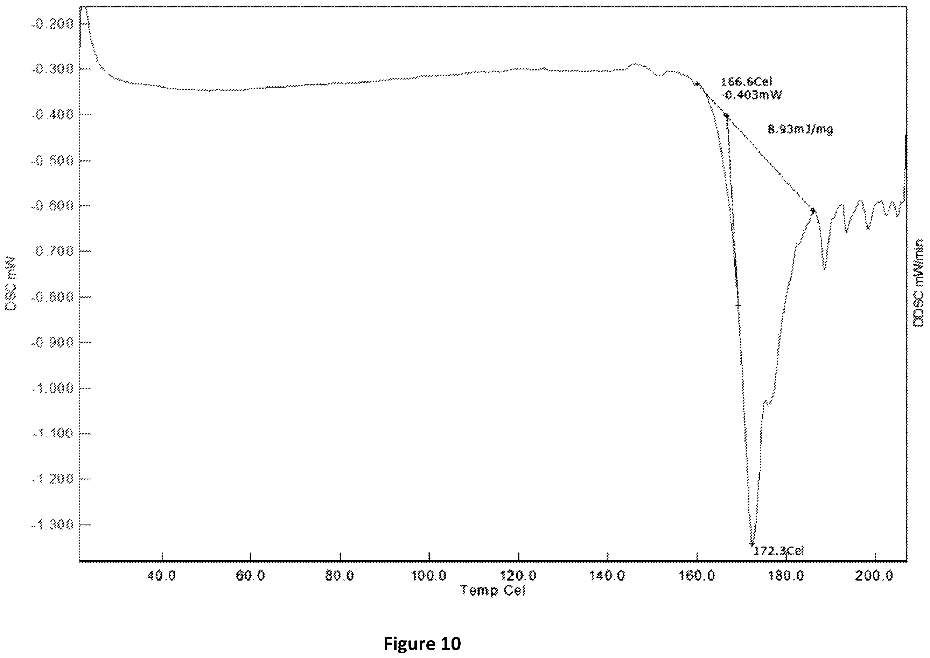

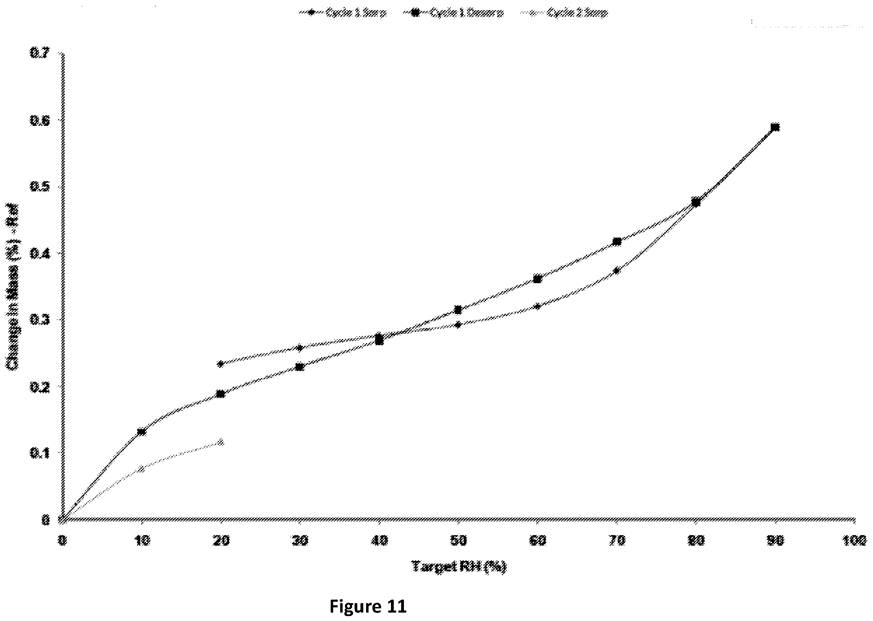

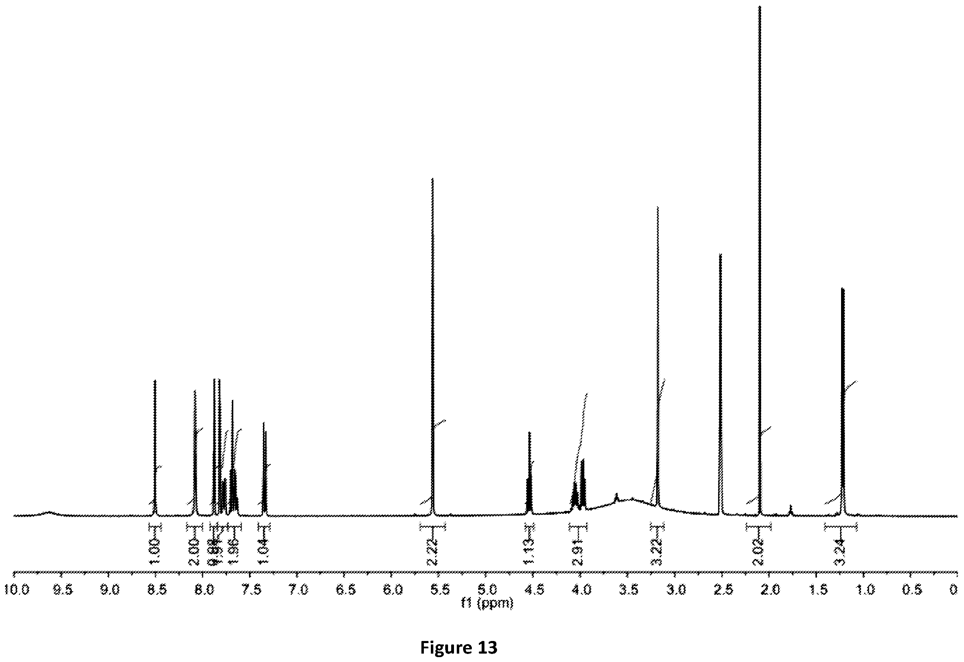

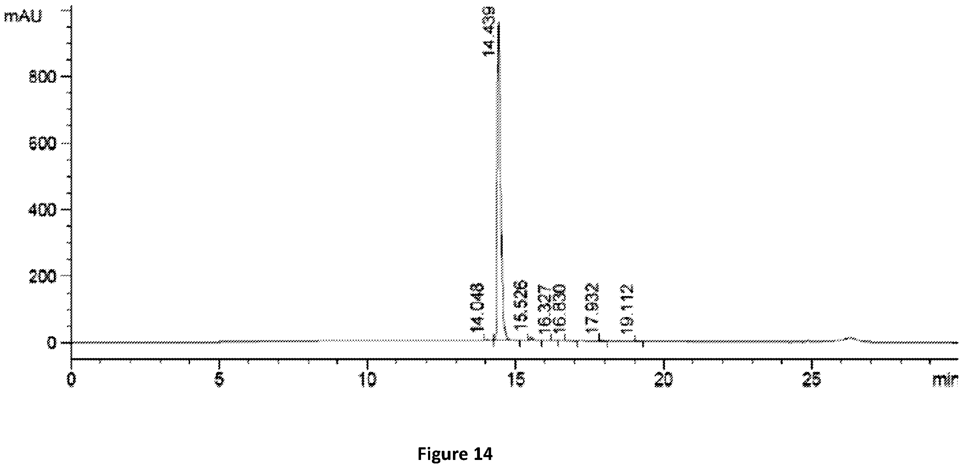

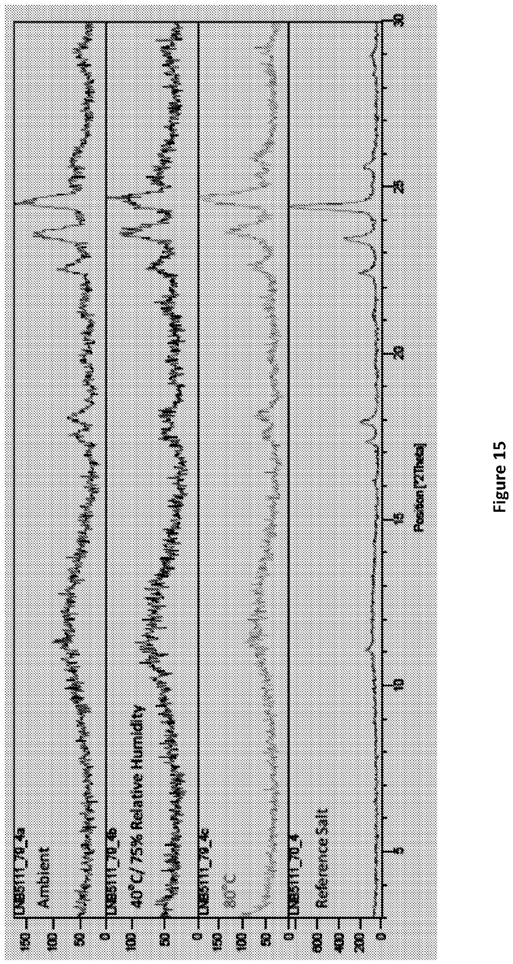

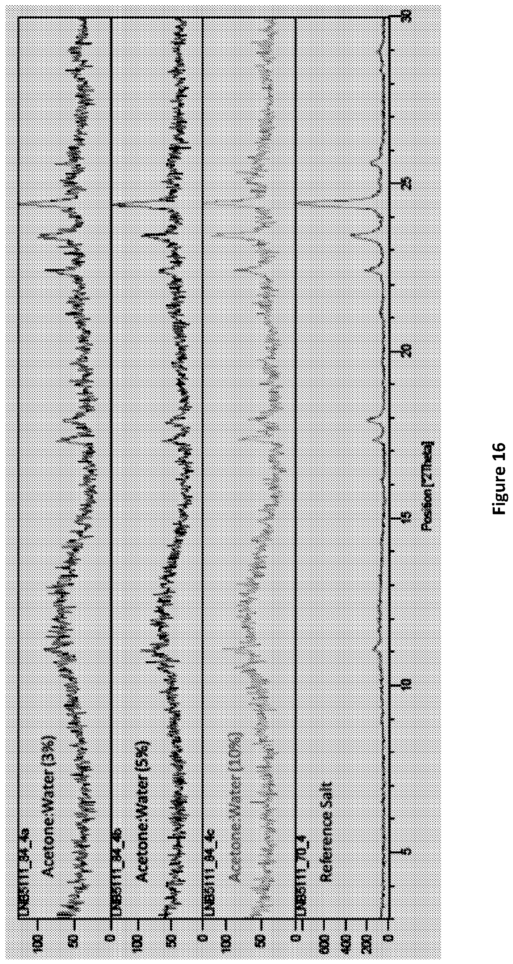

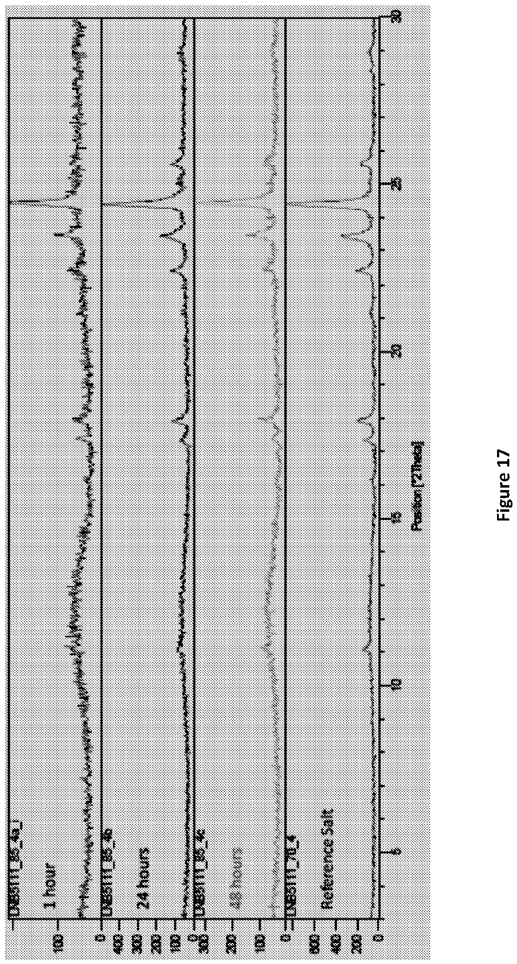

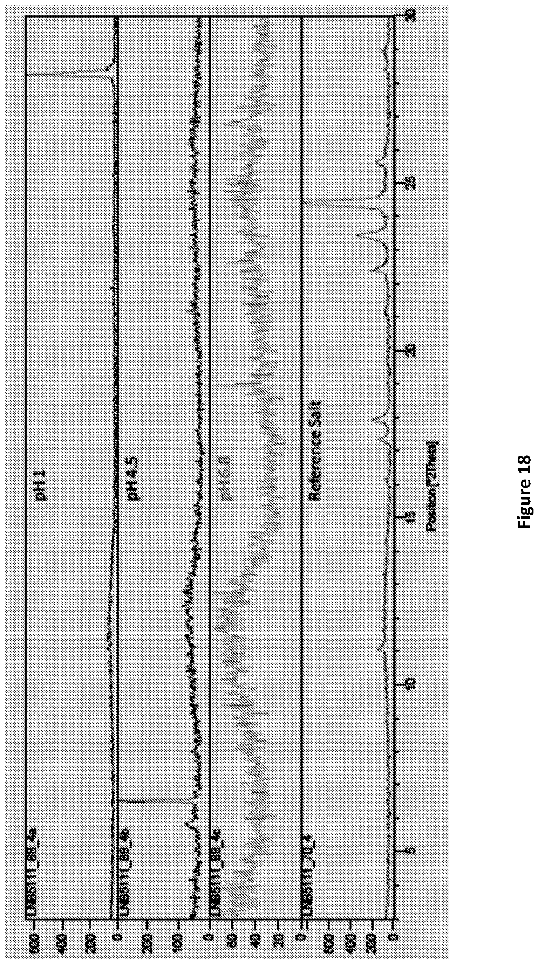

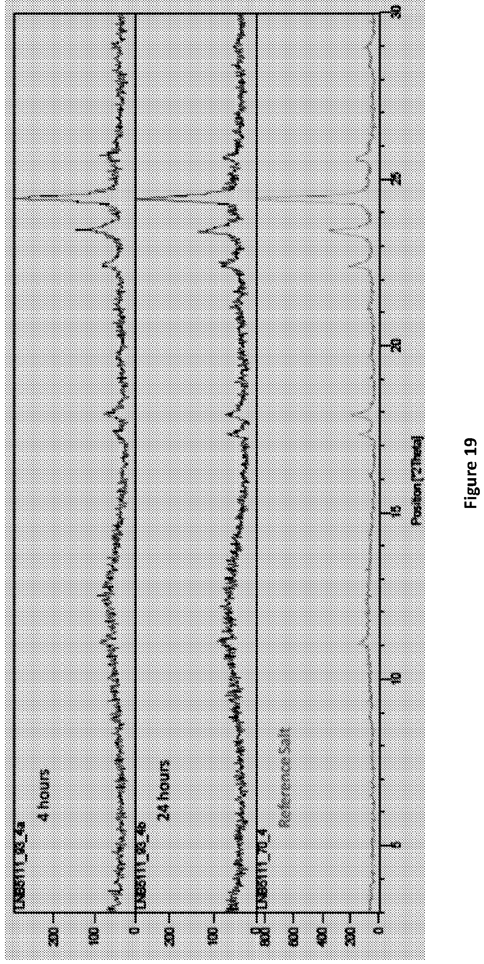

The malonate salt was prepared in acetone, using the procedure detailed in Example 3. The results can be seen in Figures 7 to 19.
- XRPD analysis shows the material to be crystalline and of the same form as produced during the primary screen.
- NMR analysis confirmed salt formation but integration is not accurate because the peak corresponding to malonic acid is positioned very close to the broad peak of water.
- Purity analysis by HPLC showed a purity of 97.99%, slightly higher purity than that of the starting material (97.82%).
- TG/DTA shows no thermal events <150°C.
- DSC analysis shows no thermal events <150°C. The onset of degradation was observed at *ca*. 180°C.
- KF analysis showed the material to contain 0.7% water.
- DVS analysis showed the material to uptake 0.14% water between 20-70% RH and 0.35% water overall. No change to the solid form was observed when analysed by XRPD, post DVS.
- Storage testing showed the material to remain unchanged during all 3 types of storage: ambient light, 40°C/75%RH and 80°C.
- Salt disproportionation studies showed there to be no form change of the malonate salt on slurrying in water.
- Hydration studies (i.e. slurry in Acetone:Water mixtures (3, 5 and 10% water)) showed the material to remain unchanged after slurrying, indicating a low hydration risk.
- Thermodynamic solubility results showed:
   - pH1 0.7687 mg/ml
   - pH 4.5 0.0028 mg/ml
   - pH 6.8 0.0020 mg/ml

A change in solid form was observed by XRPD from pH1 and pH4.5, indicating potential salt formation.
- Aqueous solubility results showed the following:
   - 4 hours 0.4947 mg/ml
   - 24 hours 0.6036 mg/ml

The material was observed to remain unchanged after slurrying. unchanged after slurrying.

### EXAMPLE 7 - Results of Purity analysis by HPLC

The results of the purity analysis by HPLC are shown in Table 6 below.

**Table 6 - HPLC purity analysis of potential salts**

| | | **Aslan001** | **Adipic Acid** | **Galactaric Acid** | **Maleic Acid** | **Malonic Acid** |
|---|---|---|---|---|---|---|
| | | **CS/245/11** | **LNB5111/70/1** | LNB5111/70/2 | **LNB5111/70/3** | **LNB5111/70/4** |
| **RRT** | **Name** | **Area %** | **Area %** | **Area %** | **Area %** | **Area %** |
| **0.97** | Carbamate | 0.36 | 0.36 | 0.31 | 0.68 | 0.36 |
| **1.00** | ARRY-334543 | 97.82 | 97.12 | 98.67 | 93.75 | 97.99 |
| **1.08** | Thiazoline | 1.17 | 1.09 | 1.02 | 1.17 | 1.00 |
| **1.11** | | 0.15 | | | | |
| **1.13** | | | | | 0.12 | 0.05 |
| **1.16** | Hydroxy Urea | 0.32 | | | | |
| **1.17** | | | 0.28 | | 0.48 | 0.25 |
| **1.19** | | 0.08 | | | | |
| **1.20** | | | | | 0.14 | |
| **1.23** | | 0.05 | | | 0.05 | |
| **1.24** | | | | | | 0.29 |
| **1.26** | | | | | 3.15 | |
| **1.30** | | | 0.90 | | 0.07 | |
| **1.31** | | 0.05 | | | | |
| **1.32** | | | | | 0.40 | 0.07 |
| **1.35** | | | 0.26 | | | |

Adipate salt showed slightly lower purity material than the starting material with an additional significant impurity at RRT 1.30 and additional minor impurity at RRT 1.35.

Mucate salt (mucic acid is also known as galactaric acid) showed higher purity material than the starting material with no additional impurities, and the Hydroxy Urea impurity was not detected. The increase in purity of ~0.85% was associated with a reduction in the levels of the named Thiazoline, Carbamate and Hydroxy Urea impurities, and the presence of no additional impurities.

Maleate salt showed a markedly lower purity material than the starting material with an additional significant impurity at RRT 1.26. Additional minor impurities and increased levels of some existing impurities were observed.

Malonate salt resulted in a slightly higher purity material than the starting material, with a reduction in the levels of the named Thiazoline and Hydroxy Urea impurities.

### EXAMPLE 8 - Conclusions

The adipate salt was found to be the least developable of the 4 salts tested in the secondary screen as it showed a tendency to disproportionate in aqueous conditions and was observed to be susceptible to hydration. Disproportionation is a specific type of redox reaction in which an element from a reaction undergoes both oxidation and reduction to form two different products.

The mucate salt (from galactaric acid) was not found to be a suitable candidate for further development as it was observed to disproportionate readily. However, a noteable increased in purity was observed, suggesting that this salt is a potential option as a process intermediate to provide improved purity in the free base. Thus, in one embodiment mucate is employed as a "transient" to generate a free base with high levels of purity.

The maleate salt was found to be promising candidate for further development, however significant impurity was observed at the 3% level that would require further investigation before progressing with this salt

The malonate salt was found to be the most developable salt, showing good stability throughout the tests employed and also a slight increase in purity over the free base.

### EXAMPLE 9 - Detailed Purity Analysis of malonate salt

Based on the above results, the present inventors decided to further investigate the potential of the malonate salt as an alternative drug substance to the Varlitinib free base.

### Preliminary results

First, a preliminary preparation of the malonate salt of Varlitinib free base from stage 9 was prepared from acetone using the method described in Example 3. The malonate salt was then analysed by HPLC and the results of analysis are shown in table 7 below.

**Table 7 - Purity analysis of Malonate salt**

| **RRT** | **Name** | **Varlitinib Input** | **Malonate Salt** |
|---|---|---|---|
| 0.97 | Carbamate | 0.36 | 0.36 |
| 1.00 | Varlitinib | 97.82 | 97.99 |
| 1.08 | Thiazoline | 1.17 | 1.00 |
| 1.11 | | 0.15 | |
| 1.13 | | | 0.05 |
| 1.17 | Hydroxyurea | 0.32 | 0.25 |
| 1.19 | | 0.08 | |
| 1.23 | | 0.05 | |
| 1.24 | | | 0.29 |
| 1.31 | | 0.05 | |
| 1.32 | | | 0.07 |

- The malonate salt had a purity of 97.99%, slightly higher than that of the Varlitinib starting material (97.82%).
- No thermal events <150°C, onset of degradation around 180°C (DSC), suggesting the malonate salt was relatively stable.
- KF = 0.7% water.
- The salt was non-hygroscopic and there was no change in PXRD post DVS.
- The salt remained unchanged under ambient light, 40°C/75%RH and 80°C (data not found).
- No disproportionation or hydration observed.

### Malonate salt prepared using Stage 7 Varlitinib free base

Next, the malonate salt was prepared in THF/water in two experiments (PR78 and PR79), this time using Varlitinib from Stage 7. The results are shown in table 8 below. The same Stage 7 input material was used in other experiments as a comparison. In two experiments (PR27 and PR28) the Stage 7 material was re-crystallised using an established re-crystallisation process. In a further two experiments (PR1 and PR24) the Stage 7 material was converted to the ditosylate salt using an established process.

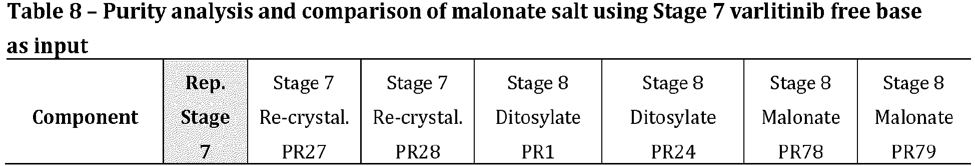

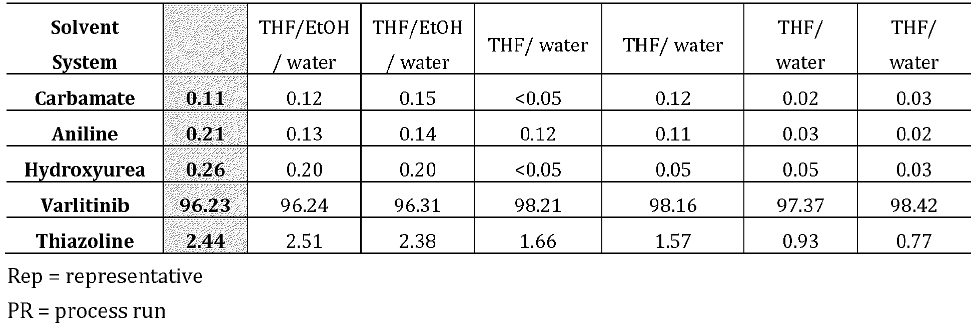

As can be seen, there was a significant decrease in aniline impurity - from 0.21 to 0.03 in PR78 and 0.02 in PR79, i.e. an approximately 10-fold decrease in aniline impurity by converting Varlitinib free base to the malonate salt thereof. This is a significant improvement compared with the existing ditosylate salt formation or the established re-crystallisation process.

Similarly, there was also a significant decrease in thiazoline levels - from 2.44 to 0.93 in PR78 and 0.77 in PR79, i.e. an approximately 3-fold decrease in thiazoline impurity by converting Varlitinib free base to the malonate salt thereof. This is a significant improvement compared with the existing ditosylate salt formation or the established re-crystallisation process.

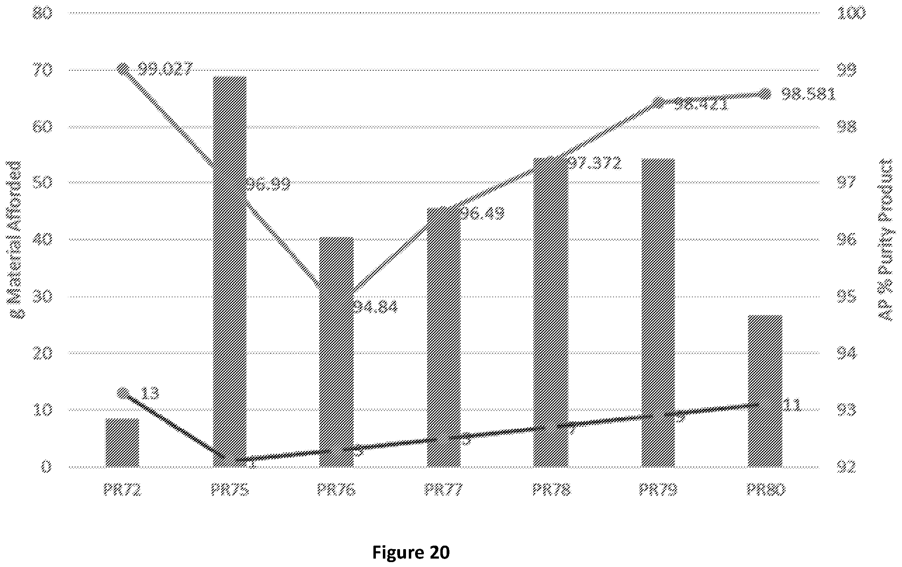

Figure 20 shows how the purity and yield of the malonate salt (generated from the free-base) is affected by the THF:water ratio. The black line shows the amount of water used (% relative to THF); the grey line shows the purity of the isolated malonate salt from each experiment; the bars show the yield of each experiment (in grams). The figure shows that the ratio of THF:water has a big impact on the yield and quality of the malonate salt, and the optimal balance was observed in experiments PR78 and PR79 in which 7% and 9% water were used respectively.

Subsequently, the malonate salt was generated from Stage 7 material using acetone/water solvent system. Data from two experiments (17/22/82 and 17/22/84) are in Table 9.

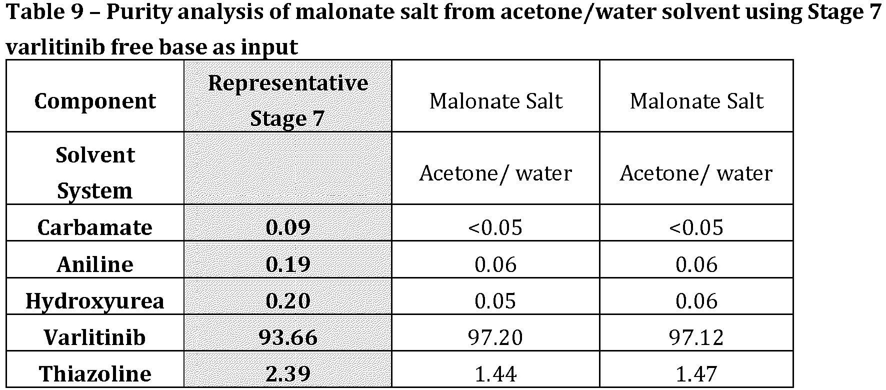

As can be seen there was a decrease in the aniline impurity from 0,19% to 0.06% in both experiments, and a decrease in the thiazoline impurity from 2.39% to 1.44% and 1.47% in both malonate processes. The acetone water solvent system purge of these impurities was superior to the existing ditosylate salt formation and the established re-crystallisation process.

Accordingly, the conversion of the Stage 7 free base of Varlitinib to its malonate salt in a range of solvent combinations results in a significant reduction in aniline and thiazoline impurity levels as compared to existing methodologies.

### Preparation of varlitinib free-base using malonate salt

Next, in order to demonstrate that the malonate salt could be successfully converted to the free-base, representative samples of the malonate salt were converted using a standard approach to Stage 9 in two experiments (ref 17/22/92 and 17/22/98). The results are presented in Table 10.

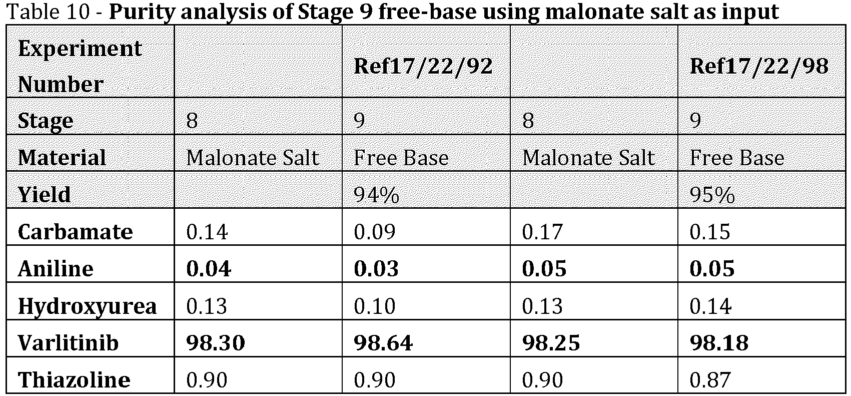

As can be seen, conversion of the malonate salt to free-base can be achieved in high yield generating API of high quality. No increase in the aniline or thiazoline impurities is observed demonstrating that the low levels of these impurities achieved during the Stage 8 salt formation could be preserved during Stage 9.

In addition, a new analytical method was developed to quantify the levels of aniline present in the drug substance in parts per million (ppm). The level of aniline in experiments 17/22/92 and 17/22/98 was quantified using this method and results are presented in Table 11.

**Table 11 - Quantification of aniline impurity in samples of free-base derived from malonate salt**

| **Experiment Number** | **Aniline Impurity (ppm)** |
|---|---|
| **17/22/92** | 323 |
| **17/22/98** | 363 |

These data are in good agreement with data reported using existing analytical methodology and confirm that the malonate salt provides greatly improved purge of the aniline impurity as compared with existing synthetic methodologies.

### Conclusions

Malonate salt appears to improve the levels of aniline impurity observed, with a maximum of 10-fold reduction observed. Malonate salt appears to offer selective purge of the thiazoline impurity.
Advantageously, the water content of the malonate salt is typically less than 1% and the salt has low propensity to form hydrates.

Furthermore, the malonate salt of Varlitinib is crystalline and the crystal form is not needles. Needles form crystals are often undesirable for pharmaceutical APIs. Varlitinib free base and the malonate counter ion form a salt in a 1:1 ratio. The salt has adequate solubility at gastric pHs and higher. The data available to date indicated the Varlitinib malonate is suitably stable and suitable for use as a pharmaceutical.

## Claims

1. A pharmaceutically acceptable salt of a compound of formula (**I**)**:** 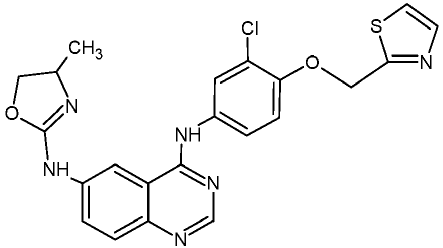 or an enantiomer thereof, wherein the salt is a malonate salt.

2. The salt according to claim 1, wherein the compound is formula (IA) is:

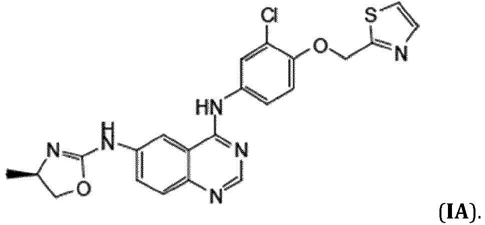

3. The salt according to any one of claims 1 or 2, wherein the purity of the salt is 97% or greater.

4. The salt according to any one of claims 1 to 3, wherein salt contains 0.1 % or less of an aniline impurity C₁₈H₁₄ClN₅OS.

5. The salt according to claim 4, wherein the aniline impurity has the structure of formula (**II**):

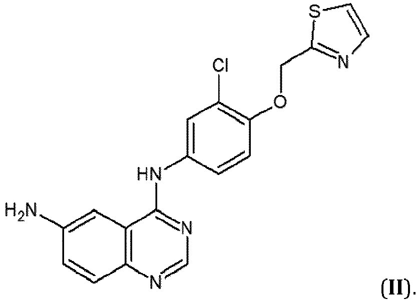

6. The salt according to claims 4 or 5, wherein said aniline impurity is present at 0.09% or less.

7. The salt according to any one of claims 1 to 6, wherein the salt contains 1.5% or less of a thiazoline impurity C₂₂H₁₉ClN₆OS₂.

8. The salt according to claim 7, wherein thiazoline impurity has the structure of formula (**III**)**:**

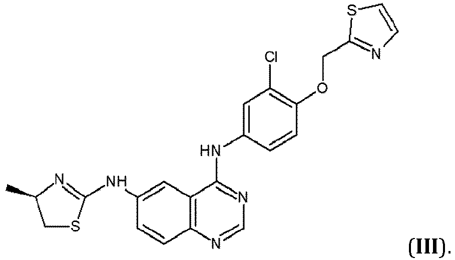

9. A pharmaceutical composition comprising a salt according to any one of claims 1 to 8.

10. A salt according to any one of claims 1 to 8 or a pharmaceutical composition according to claim 9 for use in treatment, for example for use in treating cancer.

11. A salt or composition for use according to claim 10, wherein the salt of formula (**I**) or (**IA**) is administered bi-daily.

12. A salt or composition for use according to claim 10 or 11, wherein the therapy comprises a further therapeutic agent.

13. A salt or composition for use according to claim 12, wherein the further therapeutic agent is a chemotherapeutic agent or combination thereof.

14. A salt or composition for use according to claim 13, wherein the chemotherapeutic agent is a platinum based chemotherapeutic agent selected from the group comprising cisplatin, carboplatin, oxaliplatin, satraplatin, picoplatin, nedaplatin, triplatin, lipoplatin and combinations thereof, in particular cisplatin.

15. A salt or composition for use according to claim 12 or 13, wherein the further therapeutic agent is a chemotherapeutic agent is selected from fluorouracil (5-FU), capecitabine, gemcitabine, Raltitrexed, taxol and derivatives thereof, BGC 945, OSI-7904L, FOLFOX, FOLFIRI and FOLFIRINOX, and combinations of two or more of the same.

## Patentansprüche

1. Pharmazeutisch verträgliches Salz einer Verbindung der Formel (**I**): 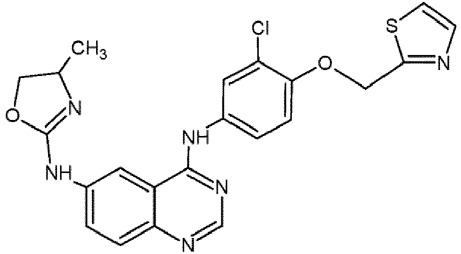 oder ein Enantiomer davon, wobei das Salz ein Malonatsalz ist.

2. Salz nach Anspruch 1, wobei die Verbindung die Formel (**IA**) aufweist:

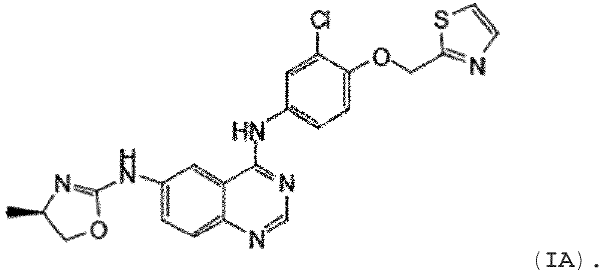

3. Salz nach einem der Ansprüche 1 oder 2, wobei die Reinheit des Salzes 97% oder mehr beträgt.

4. Salz nach einem der Ansprüche 1 bis 3, wobei das Salz 0,1% oder weniger einer Anilinverunreinigung C₁₈H₁₄ClN₅OS enthält.

5. Salz nach Anspruch 4, wobei die Anilinverunreinigung die Struktur der Formel (II) aufweist:

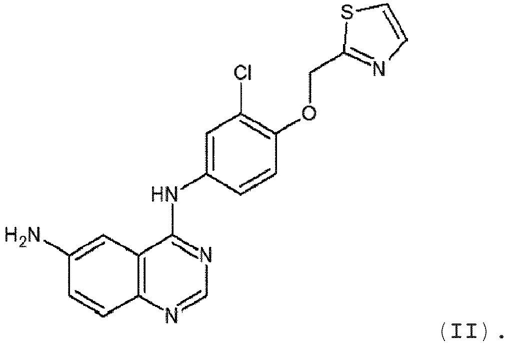

6. Salz nach Anspruch 4 oder 5, wobei die Anilinverunreinigung in einer Menge von 0,09% oder weniger vorliegt.

7. Salz nach einem der Ansprüche 1 bis 6, wobei das Salz 1,5% oder weniger einer Thiazolinverunreinigung C₂₂H₁₉ClN₆OS₂ enthält.

8. Salz nach Anspruch 7, wobei die Thiazolinverunreinigung die Struktur der Formel (**III**) aufweist:

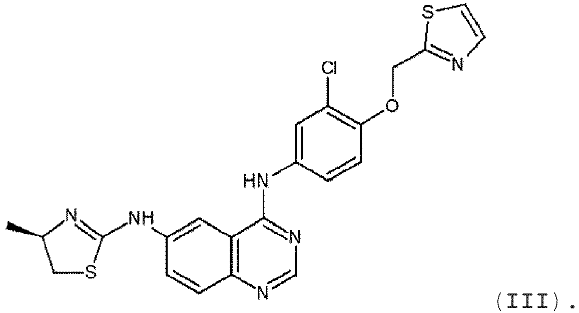

9. Pharmazeutische Zusammensetzung, umfassend ein Salz nach einem der Ansprüche 1 bis 8.

10. Salz nach einem der Ansprüche 1 bis 8 oder pharmazeutische Zusammensetzung nach Anspruch 9 zur Verwendung in der Behandlung, beispielsweise zur Verwendung bei der Behandlung von Krebs.

11. Salz oder eine Zusammensetzung zur Verwendung nach Anspruch 10, wobei das Salz der Formel (**I**) oder (**IA**) zweimal täglich verabreicht wird.

12. Salz oder Zusammensetzung zur Verwendung nach Anspruch 10 oder 11, wobei die Therapie ein weiteres therapeutisches Mittel umfasst.

13. Salz oder Zusammensetzung zur Verwendung nach Anspruch 12, wobei das weitere therapeutische Mittel ein chemotherapeutisches Mittel oder eine Kombination davon ist.

14. Salz oder Zusammensetzung zur Verwendung nach Anspruch 13, wobei das Chemotherapeutikum ein Chemotherapeutikum auf Platinbasis ist, ausgewählt aus der Gruppe bestehend aus Cisplatin, Carboplatin, Oxaliplatin, Satraplatin, Picoplatin, Nedaplatin, Triplatin, Lipoplatin und Kombinationen davon, insbesondere Cisplatin.

15. Salz oder Zusammensetzung zur Verwendung nach Anspruch 12 oder 13, wobei das weitere therapeutische Mittel ein chemotherapeutisches Mittel ist, ausgewählt aus Fluorouracil (5-FU), Capecitabin, Gemcitabin, Raltitrexed, Taxol und Derivaten davon, BGC 945, OSI-7904L, FOLFOX, FOLFIRI und FOLFIRINOX sowie Kombinationen von zwei oder mehr derselben.

## Revendications

1. Sel pharmaceutiquement acceptable d'un composé de formule **(I)** : 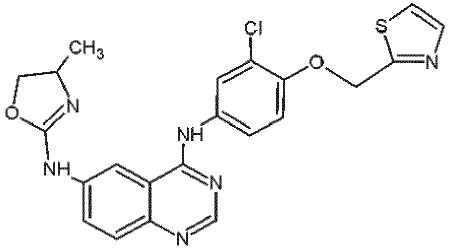 ou un énantiomère de celui-ci, dans lequel le sel est un sel de malonate.

2. Sel selon la revendication 1, dans lequel le composé de formule **(IA)** est :

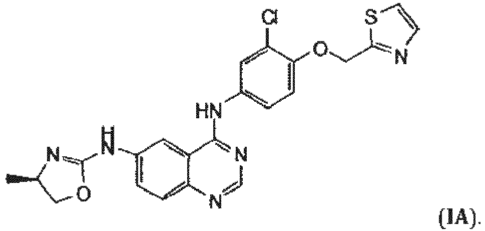

3. Sel selon l'une quelconque des revendications 1 ou 2, dans lequel la pureté du sel est de 97 % ou plus.

4. Sel selon l'une quelconque des revendications 1 à 3, dans lequel le sel contient 0,1 % ou moins d'une impureté aniline C₁₈H₁₄ClN₅OS.

5. Sel selon la revendication 4, dans lequel l'impureté aniline a la structure de formule **(II)** :

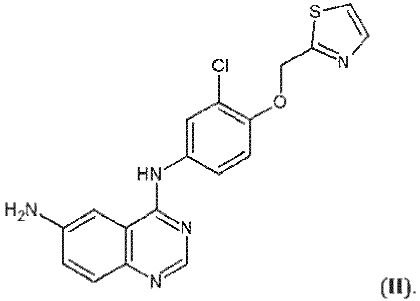

6. Sel selon les revendications 4 ou 5, dans lequel ladite impureté aniline est présente à 0,09 % ou moins.

7. Sel selon l'une quelconque des revendications 1 à 6, dans lequel le sel contient 1,5 % ou moins d'une impureté thiazoline C₂₂H₁₉ClN₆OS₂.

8. Sel selon la revendication 7, dans lequel l'impureté thiazoline a la structure de formule **(III)** :

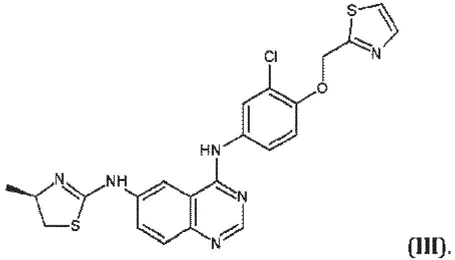

9. Composition pharmaceutique comprenant un sel selon l'une quelconque des revendications 1 à 8.

10. Sel selon l'une quelconque des revendications 1 à 8 ou composition pharmaceutique selon la revendication 9 pour une utilisation dans un traitement, par exemple pour une utilisation dans le traitement du cancer.

11. Sel ou composition pour une utilisation selon la revendication 10, où le sel de formule **(I)** ou **(IA)** est administré deux fois par jour.

12. Sel ou composition pour une utilisation selon la revendication 10 ou 11, où la thérapie comprend un autre agent thérapeutique.

13. Sel ou composition pour une utilisation selon la revendication 12, où l'autre agent thérapeutique est un agent chimiothérapeutique ou une combinaison de ceux-ci.

14. Sel ou composition pour une utilisation selon la revendication 13, où l'agent chimiothérapeutique est un agent chimiothérapeutique à base de platine choisi dans le groupe comprenant le cisplatine, le carboplatine, l'oxaliplatine, le satraplatine, le picoplatine, le nédaplatine, le triplatine, le lipoplatine et des combinaisons de ceux-ci, en particulier le cisplatine.

15. Sel ou composition pour une utilisation selon la revendication 12 ou 13, dans lequel l'autre agent thérapeutique est un agent chimiothérapeutique choisi parmi le fluorouracile (5-FU), la capécitabine, la gemcitabine, le Raltitrexed, le taxol et les dérivés de ceux-ci, BGC 945, OSI-7904L, FOLFOX, FOLFIRI et FOLFIRINOX, et des combinaisons de deux ou plusieurs des composants.
